# EUROPEAN PATENT APPLICATION

(11) **EP 1 757 261 A2**
(43) Date of publication of application: **28.02.2007**
(21) Application number: 06025888.6
(22) Date of filing: 12.07.2002
(51) Int. Cl.: A61K 8/02, C11D 17/04, A61L 15/00, A61F 13/15, A61Q 19/00

(54) **Products comprising a sheet and a lipid and aqueous phase**

(30) Priority: 13.07.2001 EP 01202691
(62) Divisional of application: 02764731.2
(71) Applicant: JOHNSON & JOHNSON GmbH, 40474 Düsseldorf (DE)
(72) Inventor: Hauser, Matthias, 53757 St. Augustin (DE); Martens, Nicolas, 53604 Bad Honnef (DE); Leonard, Mark, Bexley Kent DA5 1AZ (GB)
(74) Representative: Metten, Karl-Heinz

(57) **Abstract**

This invention concerns products for cleansing and other applications that comprise a sheet of absorbent material, in particular of non-woven material, such as a wipe, to which are applied a lipid and an aqueous phase. The invention further concerns the manufacture and use of such products.

## Description

### Field of the Invention

This invention concerns products for cleansing and other applications, which products comprise a sheet of absorbent material, in particular of a non-woven material, such as a wipe, to which a lipid and aqueous phase have been applied. The invention further concerns the manufacture and use of such products.

### Background of the Invention

Wipe products have become an important product category that has found a wide variety of applications for adults and babies. Examples include face or body cleansing wipes, wipes for skin treatment, and skin conditioning wipes. Over the last couple of decades so-called wet wipes have become successful as products particularly suited for these applications. These products typically are manufactured by impregnating sheets made of non-woven fabric with a suitable lotion.

Recent innovations in the wipes area included improvements in the fabric, in the impregnating liquid as well as in product presentation.

Initially, wet wipe products were made of traditional non-woven materials based on paper making technology (pulp based products). These products were well accepted but deficient in softness of the fabric material. The introduction of the 'spunlace' non-woven technology offered products that, compared to traditional paper based products, were superior in terms of softness. This is mainly due to (i) the use of long soft fibres (most frequently rayon and PET / PP or a mixture of these fibres) in the spunlace process and (ii) the fact that during the spunlace process no binder is added to the fabric.

Another innovation was the introduction of the so-called 'Pop-up' technology that offered advantages as regards the dispensing of individual wipes.

In addition to the above lotions have been developed which offered skincare benefits in addition to the basic cleansing properties of the wipe. One approach was the introduction of lotions that were based on oil-in-water emulsions which delivered useful properties such as superior mildness, moisturisation, protection and skin smoothness, when compared to simple aqueous cleansing formulations. Another approach encompassed the incorporation of active skincare ingredients, e.g. chamomile, into simple aqueous formulations, thereby delivering useful properties such as soothing. Current wet wipe products still are based on these approaches in that they are impregnated with either aqueous lotions or with oil in water emulsions.

However, these approaches have several limitations. Firstly, only a small portion of the lotion (usually about 15%) is released from the wipes during use. Thus a large quantity of the relatively expensive lotion is not delivered to the skin providing no benefit to the consumer and is wasted when the product is discharged after use. This also prevents the use of expensive but more effective ingredients. Secondly, from a formulation point there is an apparent contradiction in the optimization of cleansing performance and skincare benefits in one single lotion, since ingredients which are effective in cleansing usually are not compatible with efficient skin care agents.

Another important factor in cleansing is the fact that a number of soils are water-compatible and therefore more easily removed by water-based formulations, whereas others are lipid-compatible and therefore adequately removed by lipid or oil based formulations. A complete and effective removal of soils therefore requires the presence in or on a wipe of as well water and oil-based components.

This is in particularly required in wipes for personal cleansing and in particular in wipes used for babies and infants. In the latter instance wet wipes are used for cleansing the perianal region when changing diapers. Inadequate cleaning not only results in personal discomfort but also gives rise to diaper rash and other infection related phenomena. It has been shown that the most effective way of preventing diaper rash is to cleanse the skin thoroughly and to remove the microorganisms that have been identified as causative. The source of these microorganisms is often the fecal deposits that can remain on a baby's skin while wearing the diaper. Because fecal deposits consist of both water-soluble and oil-soluble matter, however, complete removal of fecal deposits from the diaper area requires both water-based and oil-based cleansing agents.

Thus, it is an object of this invention to provide a mechanism for cleansing babies' skin in order both to remove waste deposits and to reduce the number of microorganisms available to cause infection.

WO 96/14835 discloses dry tissues to which a water-in-lipid emulsion has been applied and WO 99/25923 concerns a process and an apparatus for selectively coating a wipe with a water-in-lipid emulsion. WO 99/01536 discloses wipes wherein the carrier comprises two regions of different basis weight being applied with an emulsion comprised of a solidified lipid phase, a polar phase dispersed therein and an emulsifier. Other prior art in this field is WO 95/35411, WO 95/35412, WO 95/16824, WO 97/30216, DE 33 09 530 and the publication of R.E. Mathis in Nonwovens World 1999, pp. 59-65.

It is an object of this invention to offer a cleansing article and in particular a wet wipe product that allows to independently optimize the cleansing and skincare attributes of the product and at the same time improves the delivery of skincare actives onto the skin during use.

It is a further object of this invention to provide products that have an improved release of the active ingredient(s) onto the skin during use.

It is still a further object of the present invention to provide a product for use as a cleansing tool that effectively and completely removes oil and water compatible soils.

Another object of this invention is to provide products for cleansing and other applications that allow convenient and quick application, are easy to carry, as well as an easier and more evenly distribution of the ingredients in or on the product. They moreover should be convenient for application on babies and children.

These objects are attained by the products according to the present invention, which comprise a sheet that contains an aqueous and a lipid phase. Whereas traditional wet wipes have been based on impregnation of a fabric with one phase, the products of this invention concern the application of two distinctly different phases onto a sheet. Both phases differ in terms of physical properties and may be applied on various parts or portions of the sheet. This approach allows combined optimal cleansing performance and superior skincare properties.

### Summary of the Invention

This invention relates to products that comprise a porous or absorbent sheet whereto a lipid and an aqueous phase have been applied. Preferably, the lipid phase is solid or semi-solid at ambient temperature and preferably is present at the surface or at the surface portion of one or both sides of the sheet.

In particular said sheet is made of a non-woven material, more in particular a non-woven material made by the spunlace or the hydro-entanglement procedure.

In a further aspect there is provided a method of manufacturing a product as described herein, said method comprising applying to the sheet a lipid phase and an aqueous phase, either subsequently or simultaneously. In a preferred method of manufacturing, said sheet is first coated with a lipid phase and subsequently sprayed or impregnated with an aqueous phase.

In still a further aspect there is provided the use of a product as described herein as a cleansing tool, in particular in personal care applications.

In another aspect the invention concerns the use of a product as described herein as an applicator of active substances.

In still another aspect the invention provides the use of a product as described herein as a combined cleanser and applicator of active substances.

### Detailed Description of the Invention

The absorbent or porous sheet can take the form of a tissue, a wipe, towel, towelette, and the like. The material may be flushable. As used herein, by 'flushable' is meant that the material will pass through at least 3 meters of waste pipe in two toilet flushes. The material may also be biodegradable.

Materials of which the sheet is made may be mono or multi-layered, woven or non-woven. They can be made of one or of several materials. Particularly preferred are non-woven materials that have a web structure of fibrous or filamentous nature, in which the fibres or filaments are distributed randomly or with a certain degree of orientation, the former being obtainable by air-laying or certain wet-laying processes, the latter in certain other wet-laying or in carding processes. The fibres or filaments can be natural, for example wood pulp, wool cotton, linen and the like, or synthetic, for example polyvinyls, polyesters, polyolefins, polyamides and the like.

Multi-layered sheet materials have two or more layers of the same of different materials, woven or non-woven, or layers obtained by different techniques. One embodiment is a material composed of three layers, e.g. polyethylene /pulp/polyethylene or viscose/polypropylene/viscose.

Typically the sheets have a weight per square meter in the range of 10 to 80 g/m², in particular of 20 to 70 g/m². Particular materials are of the non-woven type. Based on the raw material that has been used, two different types of products can be distinguished.

A first type of carriers is paper based. The raw materials for these carriers are made almost exclusively of cellulose-based fibres or filaments from plant cellular sources (pulp). These can be available from fresh wood-shavings or from recycled material (recycled paper). In a number of wipe applications, such as baby wipes, wipes for cleansing, wet paper towels and the like, high wet strength or firmness of the non-woven web is a desirable attribute. This can be achieved by the addition of binding materials. Examples of such materials are the so-called wet strength resins. In some cases additives are added in order to increase the softness of the end product.

In a second type use the web is made mainly of staple fibre, e.g. based on cotton, wool, linen and the like.

Commercial products are made of cellulose fibres, synthetic fibres or mixtures of both. Polyester and polypropylene are known as suitable polymers for the preparation of synthetic fibres. Also in these products binders can be used to increase the firmness of the non-woven fabric.

Webs of increased strength can be obtained by using the so-called spunlace or hydro-entanglement technique. In this technique the individual fibres are twisted together so that an acceptable strength or firmness is obtained without using binding materials. The advantage of the latter technique is the excellent softness of the non-woven material.

Non-woven materials that are made of a mixture of pulp and staple fibre are also known. Such materials are available with binding materials, in particular those mentioned above, or without binding materials. In the latter instance the non-woven is preferably made by the spunlace or hydro-entanglement procedure.

In a preferred embodiment of the present invention, the sheet material is made of cellulose pulp with a small amount of binding material. The amount of binder in the sheet material is in the range of 5 to 20 % (w/w).

In a particularly preferred embodiment the non-woven sheet material is prepared by the water entanglement procedure and does not contain binding material.

The absorbing ability of the sheet material is of particular interest with regard to the applications envisaged by the present invention. During production the impregnating solution should be taken up quickly by the sheet. In certain embodiments of this invention the wipes will be packed in a stack of a plurality of wipes. In this instance the absorbing ability of the non-woven fabric should be such that a chromatographic effect (sinking down of the lotion) in the stack is avoided during storage. On the other hand it should be guaranteed that during the usage of the wipe the impregnating solution is delivered evenly to the skin and the active ingredients are released quantitatively.

The absorbing capacity of the sheet material is determined essentially by three different parameters: the surface weight of the sheet material, the nature of the raw material used in the manufacture and the manufacturing process used.

For the applications according to the invention the sheet materials typically have a surface weight from 10 g/m² to 80 g/m², preferably from 30 to 70 g/m² and more preferably from 40 to 60 g/m². The selection of the raw material of which the non-woven sheet material is made depends on the manufacturing procedure. Typically in the manufacture of non-woven sheets by the hydro-entanglement process, use is made of mixtures of cellulose fibres and synthetic fibres. The relative quantity of synthetic fibres in the non-woven fabric is from 0 to 100 % and preferably is between 10 and 70 %, more preferably in the range of 30 to 50 % (all percentages being w/w).

According to this invention the sheet material is contacted with a lipid and an aqueous phase. In some embodiments the sheet is contacted with a third phase which may be a polymeric phase.

The phases may be applied to the whole sheet, i.e. continuously, or to parts of the sheet, i.e. discontinuously. One phase may be applied continuously while the other is applied discontinuously. They can be applied at the surface or in the internal of the sheet. If applied at the surface, one or both phases can be present at one side or at both sides of the sheet, or one phase may be present at one side while the other phase is present at the other side of the sheet.

In the instance where a phase or both phases are applied discontinuously, they are present in or at certain areas, in particular in or at one or more areas of the sheet. In that instance, the phase or phases may be present as one or more forms or shapes. For example they can be present as dots or spots, lines or stripes, as geometrical figures such as squares, rectangles, circles and the like, as symbols such as letters, text, logos, figures and the like, or as trademark signs, or any other such forms, or a combination thereof. The forms or shapes may be present over the entirety of the sheet or grouped in one or more areas, for example in a corner or in the center area.

In a particular embodiment, one phase is applied on one or on both sides of the sheet in the form of stripes, dots or other forms covering the entire surface or only a part of the surface of the sheet. The aqueous phase is applied to the sheet either on the entire surface of the fabric or on certain areas.

This may be done in a second step preferably after the application of the lipid phase or simultaneously in a one step operation.

In a preferred embodiment, both phases are applied subsequently to the sheet, more preferably first the lipid phase and subsequently the aqueous phase.

### The lipid phase

The lipid phase that is applied to the sheet is such or formulated such that it is insoluble or essentially insoluble in the aqueous phase. However, in some embodiments the two phases may be mixable or soluble into each other to a limited extend. The lipid and aqueous phase should be such or formulated such that once on the sheet and for the time prior to usage of the sheet product by the consumer they do not form one phase or a continuous phase.

In a particular embodiment, the lipid phase is hydrophobic and is composed of materials that are generally insoluble in water such as oils or fats, or waxes.

The lipid phase can be liquid, semi-solid or solid at ambient temperature. The lipid phase can be semi-solid, the latter term having the standard meaning used in the art.
It can be amorphous, semi-crystalline or crystalline, or it can take the form of a cream or waxy composition.

Semi-solidness can occur when the lipid phase is in a transition stage between solid state and liquid state such as in a melting process, but can also be due to increased viscosity of the material that makes up the lipid phase.

Semi-solidness is present in materials having a waxy, creamy, pasty, gelly or similar constituency. Semi-solidness in particular occurs with materials that have no sharp melting point, i.e. materials that have a melting range. It is also present in glass-like materials, e.g. in polymers that occur as in a glass-like state.

The lipid phase has a melting point or a melting range above or equal to 25 °C, for example in the range of 25 to 100°C, in particular in the range of 30 to 75°C, more in particular of 30 to 45°C, preferably in the range of 32 and 40°C. More preferably the melting temperature or melting range is above human body temperature. Most preferably the melting temperature or melting range approximates or is equal to human body temperature.

In some embodiments of this invention the lipid phase may have a relatively higher melting point or range. The melting point or range may for example be higher than body temperature, e.g. higher than 40 °C, or higher than 45 °C. Upon application of such products, a more intense interaction between the two phases may be required, or the application of higher temperatures, to promote the interaction. In the latter instance the consumer may, for example, be required to contact the product first with hot water and to then apply it. In the former instance the aqueous phase may contain agents that promote a stronger interaction with the lipid phase.

As used herein the term 'melting range' refers to a temperature range that starts from the temperature at which a substance or composition loses its solid constituency up to the temperature where it becomes completely liquid. A melting range is considered to be within a defined temperature range when it overlaps with that defined temperature range, or should be considered to be above a specified temperature when the range is above said temperature.

As used herein 'ambient temperature' refers to a temperature that is in the range of about 20 to about 25 °C.

The lipid phase can change to another state after application to the sheet or when being applied to the sheet during storage, or upon usage by the consumer. The lipid phase may be applied to the sheet as a liquid where after it becomes semi-solid or solid. Or the lipid phase may become semi-solid or liquid during usage by the consumer. This change of state may be induced by physical factors such as temperature or pressure but may also be induced by chemical factors such as particular components that cause a polymerization reaction or by a photochemical reaction.

In an embodiment, the lipid phase may be applied as two separate phases which become mixed during application on to the sheet, whereupon certain components in each phase become mixed and start to interact, e.g. in a polymerization reaction thus changing the state of the lipid phase from liquid to semi-solid or solid.

Particularly preferred are the compositions of the lipid phase which are solid at room temperature and which have a penetration value of 0.2 - 4 mm (measured with:
Petrotester PNR 10, Mikrokonus, 5 sec., temp 20 °C).

The water content of the lipid phase is low, in particular less than 10 %, preferably less than 6 %, more preferably less than 3 %. In a particular embodiment the lipid phase is water free, and will be such that it is not decomposed by the aqueous phase. As used herein, 'water free' means that the phase is composed of materials of low water content to which no water has been added.

The lipid phase may comprise one or more components selected from oils or fats, or waxes. It may further contain other components. As used herein oils or fats refer to the same type of materials, oils being liquid at ambient temperature and fats being solid or semi solid at ambient temperature.

The lipid phase may also comprise mixtures of waxes and fats and/or oils.

In a preferred embodiment, the lipid phase is a wax-based composition, wherein the term 'wax' is as specified hereinafter.

In particular embodiments, multiple lipid phases, i.e. lipid phases of different composition, may be applied to the sheet. For example one type of lipid phase is applied to one side of the sheet while another type is applied to the other. Each of these lipid phases may or may not contain one or more of the ingredients mentioned hereinafter, for example one or more ingredients selected from the active ingredients, the dyes, emulsifiers, and other ingredients mentioned hereinafter. In case of various dyes, multi-colored patterns may exist, for example, each lipid phase may have a different color or may be uncoloured.

The different lipid phases may be applied differently at each side of the sheet. For example one side may completely be covered while at the other side the lipid phase is applied in a pattern, e.g. as stripes.

### Oils and fats

The lipid phase may contain oils, fats or mixtures of fats with oils and/or with oily components. The resulting mixture of which the lipid phase is composed should preferably be selected in such way that the melting point or melting range of the lipid phase is as mentioned above, in particular is above ambient temperature, more in particular is in the range of 32 °C to 40 °C.

Oils or fats which can be used in the lipid phase comprise natural oils or fats, or natural oil or fat derivatives, in particular of vegetable origin. Examples are almond oil, soybean oil, sunflower oil, safflower oil, corn oil, kernel oil, canola oil, borage oil, evening primrose oil, grapeseed oil, wheat germ oil, avocado oil, jojoba oil, sesame oil, walnut oil, linseed oil, palm oil, olive oil, macadamia oil, castor oil, rapeseed oil, peanut oil, coconut oil, and turnip seed oil, and the hardened derivatives thereof. The latter are obtained by hydrogenation of fats or oils. Preferred are hardened oils or fats from vegetal origin, e.g. hardened castor oil, peanut oil, soya oil, turnip seed oil, cotton seed oil, sunflower oil, palm oil, kernel oil, linseed oil, almond oil, corn oil, olive oil, sesame oil, cocoa butter, shea butter and coconut oil.

Said hardened fats or oils have the additional advantage of increasing the constituency of the lipid phase compositions.

The lipid phase may further comprise fatty components isolated from these natural oils, i.e. pure triglycerides or mixtures thereof, or the latter components having been prepared chemically. These so-called trigycerides (or triacyl glycerines) are esters of glycerines with fatty acids or fatty acid mixtures, for example so called technical mixtures obtained by hydrolysis from fractions of oils or fats, or by fractioning fatty acid mixtures after hydrolysis. The triglycerides may also be obtained chemically by synthesis.

The fatty acids in said triglycerides may be saturated or unsaturated, straight or branch chained, substituted or unsubstituted. Preferred triglycerides are those glycerines esters derived from fatty acids, either saturated or unsaturated, having from 10 to 60, in particular from 12 to 36, more particularly from 12 to 24, preferably from 16 to 20 carbon atoms. Preferred such fatty acids are, for example, palmitic, palmic, oleic, lauric, myristic, stearic, hydroxystearic, behenic acid, or mixtures thereof. Within this group the triglycerides derived from saturated fatty acids are of particular interest.

Of particular interest are glyceryl tristearate, also referred to as stearin, glycerine tribehenate, glycerine tripalmitate, glycerine trilaurate, glycerine trioleate, glycerine trimyristate.

The lipid phase may also contain mono- or diglycerides, optionally in a mixture with the fats and oils mentioned herein, in particular with triglycerides. The mono- or diglycerides for use in the lipid phase are derived from saturated or unsaturated, linear or branch chained, substituted or unsubstituted fatty acids or fatty acid mixtures. Also in this instance the melting point or melting range of the lipid phase preferably is as mentioned above, in particular is above ambient temperature, more in particular is in the range of 32 °C to 40 °C. Particular mono- or diglycerides are mono- or di-C₁₂₋₂₄ fatty acid glycerides, specifically mono- or di-C₁₆₋₂₀ fatty acid glycerides, for example glyceryl monostearate, glyceryl distearate. Mixtures of mono-, di- and, optionally, triglycerides can be derived from fractions of fatty acids. An example of such mixture for use as a component of the lipid phase is a mixture of C₁₂₋₁₈ mono-, di- and triglycerides.

In a preferred embodiment according to the present invention the lipid phase contains one or more fatty acid glycerides selected from the mono-, di- or triesters from glycerine, or a mixture thereof.

The glycerides can be present in various amounts, it is typically present in an amount of up to 60% or in certain embodiments up to 70 %, or up to 80 % (w/w), relative to the total quantity of the lipid phase.

In other embodiments, in particular those containing dialkyl(ene)ethers or -carbonates, dicarboxylic acids or hydroxy fatty alcohols, the amount of said fatty ester glycerides will be up to 50 % and more preferably up to 40 % (w/w), relative to the total quantity of the lipid phase.

In a particular aspect of this invention there are provided products as specified herein wherein the lipid phase consists essentially of one or more fatty acid glycerides selected from the mono-, di- or triesters from glycerine, or a mixture thereof. The glyceride can be present in various amounts, e.g. the amounts mentioned hereinabove or hereinafter.

Mixed esters as well as mixtures of mono-, di- and triglycerides are of particular interest because of their low propensity to crystallize and their capacity to improve the constituency of the formulation making up the lipid phase.

The lipid phase may also comprise alkyl esters of fatty acids, wherein the alkyl group has from 1 to 30 carbon atoms, preferably from 12 to 24 carbon atoms. The fatty acids in said alkyl esters in particular are C₁₂₋₃₀ fatty acids, more in particular C₁₂₋₂₀ fatty acids. The alkyl groups in said esters preferably are derived from fatty alcohols as well as of mixtures thereof, which, for example, are obtained by high pressure hydrogenation of technical mixtures of the methyl esters derived from fats or oils.

Preferred are the alkyl esters of C₁₆₋₂₄ fatty acids, more preferably from C₁₆₋₁₈ fatty acids, and C₁₋₃₀ fatty alcohols, preferably C₈₋₂₄ fatty alcohols, more preferably C₁₂₋₂₀ fatty alcohols.

Of particular interest in this regard are, e.g. stearyl stearate, palmityl stearate, stearyl behenate, cetyl stearate, cetyl behenate, cetyl palmitate, cetearyl behenate, behenyl behenate, stearyl heptanoate, stearyl octanoate, myristyl myristate, myristyl isostearate, myristyl oleate, cetyl isostearate, cetyl oleate, stearyl isostearate, stearyl oleate, isostearyl myristat, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl isostearate, behenyl oleate, erucyl isostearate.

Of further interest are esters of linear C₆-C₂₂-fatty acids with branched alcohols, in particular 2-ethylhexanol, esters of branched C₆-C₂₂-fatty acids with linear alcohols, esters of C₁₈-C₃₈-alkylhydroxycarbonic acids with linear or branched C₆-C₂₂-fatty alcohols, esters of linear and/or branched fatty acids with poly-alcohols (e.g. propylene glycol, dimerdiol or trimertriol) and/or Guerbet alcohols, as well as esters of C₆-C₂₂-fatty alcohols and/or Guerbet alcohols with aromatic carbonic acids, in particular benzoic acid, esters of C₂-C₁₂-dicarbonic acids with linear or branched C₁-C₂₂ -alcohols (e.g. dioctyl malate) or C₂-C₁₀-polyoles having 2 to 6 hydroxyl groups.

Preferred fats comprise the triglycerides, in particular those derived from fatty acids having from about 12 to about 24 carbon atoms, in particular those having from about 12 to about 20 carbon atoms, more in particular those having from about 16 to about 20 carbon atoms. These fatty acids may be unsaturated or, which is preferred, saturated.

Particularly preferred are glycerides derived from oleic, stearic, myristic or lauric acid, or from fatty acid mixtures derived from natural oils such as coco-acids. Examples of preferred fats are cocoglycerides, glyceryl stearate, glyceryl laurate, and the like.

Further preferred fats comprise hydrogenated natural oils such as hydrogenated castor oil, hydrogenated palm oil and the like.

The lipid phase may also comprise oily components, i.e. non water-mixable components that are liquid at 20 °C. These can be e.g. glycerides, hydrocarbons, silicon oils, ester oils and the like, as well as mixtures thereof. The total quantity of these oily components in the total composition of the lipid phase preferably will be such that the lipid phase is solid at room temperature, or that it has a melting point or range that is as specified hereinabove. The oily components will typically be present in quantities of less than 40 % (w/w), in particular less than 20 %, or further in particular 1- 15 %, more in particular from 2 - 10 % (w/w) relative to the total quantity of the lipid phase.

The oily components can be any of the oils mentioned hereinabove as 'oils and fats', more in particular the mono-, di- and triglycerides mentioned hereinabove, that are liquid at 20 °C. The oily components can further be fatty acids and fatty alcohols, described hereinabove in the respectively sections, therein that are liquid at 20 °C.

Further oily components which can be used in the lipid phase comprise silicone oils, mineral and paraffin oils and synthetic oils, either aliphatic or aromatic, as well as mixtures thereof. Examples of such oils are squalane, squalene, isohexadecane, isoeicosane, polydecene, and also members of the group of dialkylcyclohexanes.

The lipid phase may further contain silicone oils such as, for example cyclic silicones, dialkyl- or alkylarylsiloxanes, e.g., cyclomethicone, dimethyl polysiloxane and methylphenyl polysiloxane, as well as the alkoxylated and quaternized analogs thereof. Appropriate non-volatile silicon oils are e.g. polyalkylsiloxanes, polyalkylarylsiloxanes and polyethersiloxane-copolymers.

The total amount of fats or oils, or of mixtures of fats and oils and/or oily components in the lipid phase in particular is at least 50 %, preferably at least 70 %, more preferably at least 90 %, w/w of the total amount of components making up the lipid phase.

In a particular aspect of this invention there are provided products as specified herein wherein the lipid phase essentially consists of fats or oils, or of mixtures of fats and oils and/or oily components, in particular those specified in this specification. The fats, oils and oily components can be present in various amounts, e.g. the amounts mentioned hereinabove or hereinafter.

### Waxes

The lipid phase may comprise waxes. As used herein, the term 'wax' refers to oil soluble materials that have a waxy constituency and have a melting point or range of above ambient temperature, in particular above 25 °C. Waxes are materials that have a solid to semi-solid (creamy) consistency, crystalline or not, being of relative low viscosity a little above their liquefying point. Waxes can be composed of one or more components, synthetic as well as natural, and can in principle be composed of or comprise any oil soluble material having a waxy constituency, including mixtures thereof.

Waxes which can be used may be synthetic or natural waxes, as well as other oil soluble materials that have a waxy consistency. Waxes also encompass materials such as oils or fats of natural or synthetic origin, and waxy components such as higher alkanols (in particular fatty alcohols), higher alkanediols (in particular hydroxy fatty alcohols) carboxylic acids (in particular fatty acids), dialkyl(ene)ethers, dialkyl(ene) carbonates, dicarboxylic acids and the like components.

Natural waxes comprise waxes from vegetal origin, such as purcelline, shea butter, cocoa butter, Japan wax, esparto gras wax, cork wax, Guaruma wax, rice shoot wax, Ouricury wax, montan wax, sunflower wax, ceresine wax, sugar cane wax, carnauba wax, candelilla wax, lanolin, fruit-derived waxes, such as orange wax, lemon wax, grapefruit wax and bayberry wax, and the like, and of animal origin such as beeswax, woolwax, spermateci and bear fat, shellac wax, and the like. Natural waxes further comprise mineral waxes such as ceresine and ozokerite waxes. Synthetic waxes comprise petroleum-based waxes such as paraffin, vaseline, petrolatum, micro wax. Further synthetic waxes are polyalkylene and polyethyleneglycol waxes, e.g. polyethylene wax; waxes based on chlorinated naphtalenes such as 'Halowax', synthetic hydrocarbon waxes, and the like, including mixtures thereof. Further waxes are chemically modified waxes, in particular hardened or hydrogenated waxes such as, for example, Montan-ester waxes, Sasol waxes and hydrogenated jojoba waxes.

Preferred among these natural waxes are waxes from vegetal origin.

Other wax components can be certain fats (including mono-, di- and triglycerides and fatty acid alkylesters), fatty alcohols, fatty acids, including substituted fatty acids (in particular hydroxy substituted fatty acids, for example, 12-hydroxystearic acid), dialkyl(ene)ethers, dialkyl(ene) carbonates, dicarboxylic acids (in particular the C₁₆-C₄₀-dialkylesters of dicarboxylic acids, e.g. the C₁₆-C₄₀-alkyl stearates, C₁₈-C₃₈-alkylhydroxystearyl stearates or C₂₀-C₄₀-alkyl erucates) and hydroxy fatty alcohols that comply with the definition of 'wax' as outlined herein. Any of these components may contain homologous components that are liquid, as long as the total composition making up the lipid phase has a waxy constituency. For example, waxy fats may contain oils, waxy fatty alcohols may contain liquid fatty alcohols, etc., in such amount that the total composition has a waxy constituency and in particular has the melting point or range specified above.

Still further wax components are selected from the group of aromatic carbonic acids, tricarboxylic acids, or from the group of lactides of long-chained hydroxycarbonic acids. Myristyl lactate is particularly attractive for use on wipes for skin treatment, because of its binding capacity to the skin.

Further wax components that can be used are C₃₀-C₅₀-alkyl bees wax; tri-C₁₆-C₄₀-alkyl citrates, e.g. tristearyl citrate, triisostearyl citrate, trilauryl citrate; ethyleneglycol di fatty acid esters, in particular the ethylene glycol di-C₁₂-C₃₀-fatty acid esters, e.g. ethylene glycol dipalmitate, ethyleneglycol distearate, ethyleneglycol di(12-hydroxystearate).

As further useful components there can be mentioned silicone waxes.

The lipid phase may also comprise mixtures of waxes and fats and/or oils.

The total amount of waxes in the lipid phase in particular is at least 50 %, preferably at least 70 %, more preferably at least 90 %, w/w of the total amount of components making up the lipid phase.

In a particular aspect of this invention there are provided products as specified herein wherein the lipid phase essentially consists of one or more waxes selected from the waxes mentioned herein, including mixtures thereof. The waxes can be present in various amounts, e.g. the amounts mentioned hereinabove or hereinafter.

### Fatty alcohols

The lipid phase may also comprise fatty alcohols. Fatty alcohols that can be used are, for example, C₁₂-C₅₀-fatty alcohols, in particular the C₁₂-C₂₄-fatty alcohols, that are derived from natural fats, oils or waxes such as, for example, myristylalcohol, 1-pentadecanol, cetylalcohol, 1-heptadecanol, stearylalcohol, 1-nonadecanol, arachidylalcohol, 1-heneicosanol, behenylalcohol, brassidylalcohol, lignocerylalcohol, cerylalcohol or myricylalcohol as well as Guerbet alcohols. Preferred for use in the present invention are saturated, straight or branch chained fatty alcohols. However also unsaturated, straight or branch chained alcohols can be used, optionally in a mixture with saturated alcohols. Preferably the alcohols will be selected such that the melting point of the mixture is as referred to hereinabove and more in particular is in the range of 32 to 40 °C.

Mixtures of fatty alcohols can evidently also be used, including fatty alcohol fractions obtained from the reduction of the corresponding fatty acid fractions derived from naturally occuring oils or fats such as, for example, almond oil, soybean oil, sunflower oil, safflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, coconut oil, and turnip seed oil.

Synthetic alcohols can also be used such as, for example, the linear fatty alcohols of an even number of carbon atoms resulting from the Ziegler-synthesis (Alfole^{®}) or the partially branched alcohols resulting from the Oxo synthesis (Dobanole^{®}).

A preferred embodiment according to the present invention is that wherein the lipid phase contains at least one fatty alcohol, more preferably at least one C₁₄-C₁₈-fatty alcohol. Also preferred is a lipid phase with at least one C₁₆-C₁₈-Guerbet alcohol.

The use of fatty alcohols advantageously results in the lipid phase having a drier, i.e. less greasy, skin feel, compared to components such as triglycerides.

The total amount of fatty alcohols in the lipid phase may vary and depends on the desired properties of the lipid phase. In a number of instances it is desirable to have a relative higher quantity of fatty alcohols in the composition, in particular said alcohols will be present in an amount of 50 %, preferably at least 70 %, more preferably at least 90 %, (w/w) of the total amount of components making up the lipid phase. In other instances, relatively lower amounts are desired, the total amount of the fatty alcohols present in the lipid phase is in the range of 1 - 40 %, preferably of 1 - 30 % (w/w), more preferably of 1 - 20 % (w/w), still more preferably from 1 -10 % (w/w).

In a particular aspect of this invention there provided products as specified herein wherein the lipid phase essentially consists of one or more fatty alcohols, in particular those specified in this patent specification, including mixtures thereof. The fatty alcohols can be present in various amounts, e.g. the amounts mentioned hereinabove or hereinafter.

### Fatty acids

The lipid phase may also contain C₁₄-C₄₀-fatty acids, including mixtures thereof. Of particular interest are the C₁₆-C₃₀-fatty acids. These comprise, for example, myristic-, pentadecanoic-, palmitic-, margaric-, stearic-, nonadecanoic-, arachic-, behenic-, lignoceric-, cerotic-, melissic-, erucaic-, elaeostearic-, oleic-, lonolenic-, lauric acid as well as substituted fatty acids, e.g. hydroxy-substituted fatty acids such as, for example, 12-hydroxystearic acid, and the amides or monoethanolamides of these fatty acids.

The total amount of the C₁₄-C₄₀-fatty acids present in the lipid phase, relative to the total weight amount of the lipid phase, is in the range of 1 - 30 % (w/w), preferably of 1 - 20 % (w/w), more preferably from 1 -10 % (w/w).

In a particular aspect of this invention there are provided products as specified herein wherein the lipid phase essentially consists of one or more fatty acids, in particular those specified in this patent specification, including mixtures thereof. The fatty acids can be present in varying amounts, e.g. the amounts mentioned hereinabove or hereinafter.

### Dialkyl(ene)ethers or -carbonates, dicarboxylic acids or hydroxy fatty alcohols

The lipid phase may also contain dialkyl(ene) ethers, dialkyl(ene) carbonates, dicarboxylic acids or hydroxy fatty alcohols, or mixtures thereof, which ethers, carbonates, acids or alcohols in particular those described hereinafter.

In a particular aspect of this invention there are provided products as specified herein wherein the lipid phase essentially consists of one or more dialkyl(ene) ethers or - carbonates, dicarboxylic acids or hydroxy fatty alcohols, including mixtures thereof. The dialkyl(ene) ethers or -carbonates, dicarboxylic acids or hydroxy fatty alcohols can be present in various amounts, e.g. the amounts mentioned hereinabove or hereinafter.

The addition of dialkyl(ene) ethers or -carbonates, dicarboxylic acids or hydroxy fatty alcohols, including mixtures thereof to the composition of the lipid phase allows to optimize the properties of the lipid phase, in particular its sensoric properties, i.e. the products as well as the skin after the products have been applied have a less greasier feel and also a less dry skin-feel, while having excellent skin caring properties.

### Dialkyl(ene) ethers

The dialkyl(ene) ethers are symmetric or asymmetric, straight or branch chained, saturated or unsaturated. Preferred are waxy, saturated C₁₆-C₃₀-dialkylethers, in particular C₁₆-C₂₄-dialkylethers. More preferred are C₁₆-C₂₀-dialkylethers, and particularly preferred are distearylethers and dibehenylethers. Dialkylethers of shorter chain length can also be used such as, for example, di-n-octylether, di-(2-ethylhexyl)-ether, laurylmethylether or octylbutylether, didodecylether, under the condition that the complete composition of the lipid phase has the desired melting point.

These ethers can be obtained from the appropriate fatty alcohols in the presence of an acid catalyst following art-known procedures. Typical examples are the products that are obtained by the etherification of capron alcohol, capryl alcohol, 2-ethylhexyl alcohol, caprin alcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol, elaeostearyl alcohol, arachidyl alcohol, gadoleylalcohol, behenyl alcohol, erucyl alcohol and brassidyl alcohol, Guerbet alcohols, as well as mixtures thereof, which, for example, are obtained by high pressure hydrogenation of technical mixtures of the methyl esters derived from fats or oils.

Of particular interest are the dialkyl(ene) ethers that are solid at 25 °C.

### Dialkyl(ene) carbonates

The dialkyl(ene) carbonates are symmetric or asymmetric, straight or branch chained, saturated or unsaturated. Preferred dialkyl(ene) carbonates are waxy, linear or branch chained, saturated or unsaturated C₁₄-C₃₀-dialkyl(ene) carbonates. More preferred are C₁₆-C₂₄-dialkyl carbonates and amongst these the saturated linear C₁₆-C₂₂-dialkyl carbonates. Particularly preferred is distearyl carbonate. Also liquid dialkyl(ene) carbonates, such as, for example, dihexyl-, dioctyl-, di-(2-ethylhexyl)- or dioleylcarbonate, can be used, under the condition that the complete composition has the desired melting point.

These dialkyl(ene) carbonates can be obtained by re-esterification of dimethyl- or diethylcarbonates with the corresponding hydroxy compounds following art-known procedures. Typical examples of dialkyl(ene) carbonates are re-esterification products of dimethyl- and/or diethylcarbonate with capron alcohol, capryl alcohol, 2-ethylhexyl alcohol, caprinalcohol, lauryl alcohol, myristyl alcohol, cetyl alcohol, palmoleyl alcohol, stearyl alcohol, isostearyl alcohol, elaidyl alcohol, petroselinyl alcohol, linolyl alcohol, linolenyl alcohol, oleyl alcohol, ricinus alcohol, elaeostearyl alcohol, arachidyl alcohol, gadoleyl alcohol, behenyl alcohol, erucyl alcohol and brassidyl alcohol, Guerbet alcohols, as well as technical mixtures thereof, that can be obtained by hydratation of methyl esters derived from suitable oils or fats or oil or fat fractions.

Of particular interest are those dialkyl(ene) carbonates that are solid at 25 °C.

### Dicarboxylic acids

Dicarboxylic acids that can be used are, for example, C₉-C₃₄-dicarbonic acids.

### Hydroxy fatty alcohols

The hydroxy fatty alcohols for use in the said preferred or particularly preferred waxy compositions are saturated or unsaturated, straight chain or branched. Preferred are C₁₂-C₃₀-hydroxy fatty alcohols, at which the position of the hydroxy-substituent depends upon the synthesis route and the starting materials that have been used. Included are, for example 1,10-decanediol, 1,2-hexadecanediol, 12-hydroxystearyl alcohol or hydroxy-Guerbet alcohols. Preferred are those hydroxy fatty alcohols that are solid at 25 °C, although liquid analogs can also be used, as long as the complete composition has the desired melting point. Particularly preferred is 12-hydroxystearyl alcohol.

The total amount of one or more of the dialkyl ethers, dialkyl carbonates, dicarbonic acids and the hydroxyalcohols present in the lipid phase, relative to the total weight amount of the lipid phase, is in the range of 1 - 30 % (w/w), preferably of 1 - 20 % (w/w) more preferably from 1 -10 % (w/w).

### Other components

The compositions of the lipid phase may contain further components, which may be of waxy nature or otherwise. The use of these further components allows to influence the sensorical properties as well as the stability of the compositions, in particular after application to wipe material and more in particular when in contact with the aqueous phase. The other components may also be added to influence constituency, feel and appearance. These components will generally be insoluble or poorly soluble in water. Water soluble components can also be included, typically in combination with a solubilizing or emulsifying agent and some water.

Examples of further components are superfatting agents, thickeners, polymers, active ingredients, film forming agents, UV-filters, anti-oxidants, hydrotropic agents, preservatives, insect repellents, self-tanning agents, solubilizers, perfume oils, dyestuffs and the like.

Substances that can be used as superfatting agents are, for example, lanolin or lanolin derivatives such as lanolin alcohols, lanolin acids, polyethoxylated or acylated lanolin, or other lanolin derivatives; phospholipids such as lecithin or lecithin derivatives such as polyethoxylated or acylated lecithin or other lecithin derivatives; polyol fatty acid esters, monoglycerides and fatty acid alkanolamides.

Appropriate thickeners for example are of the Aerosil ®-type (hydrophilic silica acids), polysaccharides, in particular xanthan-gum, guar-guar, agar-agar, alginate and tyloses, carboxymethyl cellulose and hydroxyethyl cellulose, additionally relatively high molecular weight polyethylene glycol mono- and -diesters of fatty acids, polyacrylate, (for example Carbopol^{®} of Goodrich or Synthalene^{®} of Sigma), poly-acrylamides, polyvinylalcohol and polyvinylpyrrolidone, surfactants such as, for example, ethoxylated fatty acid glycerides, ester of fatty acids with polyoles such as, for example, pentaerythrit or trimethylolpropane, fatty alcohol ethoxylates having limited range of homologs or alkyloligoglucosides as well as electrolytes such as sodium chloride ammonium chloride.

Appropriate cationic polymers are for example cationic cellulose derivatives , e.g. quaternized hydroxyethyl cellulose (commercialized under the trade name Polymer JR 400^{®} by Amerchol), cationic starches, copolymers of diallylammonium salts and acrylamides, quaternized vinylpyrrolidone/vinylimidazole-polymers (for example Luviquat^{®} of BASF), condensation products of polyglycols and amines, quaternized collagen polypeptides, such as, for example, lauryldimonium hydroxypropyl hydrolyzed collagen (Lamequat^{®}L/Grünau), quaternized wheat polypeptides, polyethylene imines, cationic silicone polymers, e.g.. amodimethicone, copolymers of adipinic acid and dimethylaminohydroxypropyldiethylenetriamine (Cartaretine^{®}/Sandoz), copolymers of acryl acid with dimethyldiallylammoniumchloride (Merquat^{®} 550/Chemviron), polyaminopolyamides, cationic chitine derivatives such as, for example, quaternized chitosans, optionally dispersed in microcristalline form, condensation products derived from dihalogenalkylenes, such as, for example dibromobutane with bis-dialkylamines, e.g. bis-dimethylamino-1,3-propane, cationic guar-gum, such as, for example, Jaguar^{®} CBS, Jaguar^{®} C-17, Jaguar^{®} C-16 from Celanese, quaternized ammonium salt-polymers, e.g. Mirapol^{®} A-15, Mirapol^{®} AD-1, Mirapol^{®} AZ-1 from Miranol.

Anionic, zwitterionic, amphoteric and nonionic polymers that can be used are, for example, vinylacetate/crotonic acid-copolymers, vinylpyrrolidon/vinylacrylate-copolymers, vinylacetate/butylmaleate/ isobornylacrylate-copolymers, methylvinylether/maleic acid anhydride-copolymers and their esters, which are not cross-linked and with polyoles linked polyacrylacids which are cross-linked, acrylamidopropyl trimethylammonium chloride/ acrylate-copolymers, octylacrylamide/methylmethacrylate/tert.butylaminoethylmethacrylate/2-hydroxypropylmethacrylate-copolymers, polyvinylpyrrolidone, vinylpyrrolidone/vinylacetate-copolymers, vinylpyrrolidone/dimethylaminoethylmethacrylate/vinyl caprolactam-terpolymers as well as optionally derivatized cellulose ethers and silicones.

As further consistency agents there can be used small amounts of alkalimetal or alkaline earth metal as well as aluminium salts of C₁₂-C₂₄-fatty acids or C₁₂-C₂₄-hydroxyfatty acids, preferred being calcium-, magnesium-, aluminium- and in particular zinc stearates.

The lipid phase may further contain suitable anti-oxidants such as, for example, sulfites, e.g. sodium sulfite, tocopherol or derivatives s thereof, ascorbic acid or derivatives s thereof, citric acid, propyl gallate, chitosan glycolate, cysteine, N-acetyl cysteine plus zinc sulfates, thiosulfatess, e.g. sodium thiosulfates, polyphenoles and the like.

The lipid phase may further contain powders or powdered ingredients or mixtures thereof such as talcum, Bolus alba, myristyl alcohol, cetyl alcohol, cetylstearyl alcohol, calcium or magnesium stearate, magnesium lauryl sulfate, starch or derivatives thereof e.g. distarch phosphate, aluminium starch octenylsuccinate, carboxymethyl starch, tapioca starch, dimethylimidazolidinone rice starch, sodium starch glycolate, potato starch, rice starch, corn starch, hydroxypropyl starch, hydroxyethyl starch and the like.

The lipid phase may further contain disintegrating agents, which are agents that cause a disintegration of the physical integrity of the lipid phase. The disintegration may be in parts or on the whole of the lipid phase. The disintegrating agents may be mixed or dissolved into parts or the whole of the lipid phase. The disintegrating agents may be mixed continuously in the lipid phase or discontinuously, e.g. at the top side of the lipid phase, e.g. where the lipid phase is applied as a layer, at the top of that layer or in the top portion of that layer.

Suitable disintegrating agents are agents that are subject to physical or chemical interactions either by auto-interaction or by interaction between two agents. This results in a physical or chemical interaction with the lipid phase. One type of disintegrating agents are those that release a gas e.g. by decomposition or by chemical reaction between two components. An example of a disintegrating agent is a solid mixture of a bicarbonate and an acid such as sodium or potassium carbonate with a suitable organic acid, e.g. citric acid. Upon contact with water, e.g. upon contact with the aqueous phase, the disintegrating components will interact and liberate carbon dioxide which physically alters the lipid phase. Such physical alteration may, for example, cause the lipid phase to become homogeneously distributed on the sheet. This may positively influence the interaction between the aqueous and lipid phases, which in turn may have a positive effect on the transfer to the skin of materials, e.g. active ingredients, in these phases.

The lipid phase may further contain components that are subject to a polymerization reaction either during or after application on the sheet material. Examples of such components are oligomers that during or after application on the sheet continue to polymerize with monomers or other oligomers. Other examples are agents that cause netting or co-polymerisation. There can also be agents that inhibit polymerization for a specific period of time. Alternatively there can be agents that accelerate polymerization e.g. under influence of external factors such as heat, light or pressure.

In one type of embodiment, the lipid phase contains monomers or oligomers that can be caused to polymerize or co-polymerize under the influence of an external factor, an example of the latter being light. The lipid phase is applied to the sheet and during the application process the lipid phase is subjected to light radiation whereupon polymerization occurs. Alternatively, the lipid phase may be subjected to light radiation after it having been applied to the sheet.

The lipid phase may further contain dyes that upon usage of the product change color due to a change of temperature or pressure. This will give the consumer a level of comfort and trust that the product delivers the lipid phase to the skin, or in case of a lipid phase containing active ingredients that the latter are delivered onto the skin.

The lipid phase may further contain dye-precursors, i.e. agents that become dyed upon influence of physical or chemical factors. In particular embodiments the lipid phase may contain dye-precursors which react with certain agents that are present in the aqueous phase so as to form a dye. Similarly, the dye-precursors may be present in the aqueous phase and become transferred into dyes upon interaction with certain chemicals incorporated into the lipid phase.

The lipid phase can also be formulated to or into beads. Particularly such beads are polymeric beads wherein the lipid phase is entrapped in whatever form. The terms 'beads' or 'polymeric beads' are meant to comprise any form of discrete, free-flowing powders, beads or capsules which envelope, coat or contain a lipid phase in a mono- or polymeric matrix or capsule. These terms also encompass powders, beads or capsules wherein the mono- or polymeric matrix itself is a lipid phase. These terms are also meant to include porous beads or 'microsponges' and 'microcapsules', the latter being beads of smaller size. The beads may be coated with a suitable coating material that protects the interior of the bead or controls the release of the lipid phase entrapped therein. The coating on the bead itself may contain a lipid phase. In the latter instance, the coating is layed on an inert core or on a core containing lipid phase and/or other ingredients.

Formulation of a lipid phase in beads may be done for protecting the lipid phase from external factors that may impact its integrity. However, it is mostly done for allowing controlled release of the lipid phase.

A particular type of beads are small beads or capsules, having an average diameter which is in the micrometer range, although the average diameter can be as small as even 200 nm.

This type of capsules can be liposome-based, made for example of phospholipids such as lecithin, phosphatidyl ethanolamine, phosphatidyl serine, phosphatidic acid and the like. This type of capsules also can be made of starch, cellulose, porous gelatin and the like.

The capsules or beads can also be relatively larger, having average sizes in the mm or 0.1 mm range. This type of capsules or beads can be made of materials such as agar, glycolic acid polymers, and further components such as water, mineral oils, glycerin. They may contain further ingredients such as preservatives, dye(s), and the like.

Another type of beads or microcapsules are microsponges. These are materials sized from about 5 to about 300 µm (average diameter) having a large inner surface. These are obtained by polymerization of particular monomers. Lipid phase material can be entrapped therein either during this polymerization process or afterwards.
Microsponge-based carriers may be used to protect the lipid phase entrapped therein or for controlled release purposes.

The capsules may optionally contain one or more suitable disintegrating agents, in particular those mentioned in this specification. Upon contact with the appropriate external factor, the disintegrating agents will cause the capsules to break open thus allowing release of the lipid phase entrapped therein.

The capsules can be incorporated into the aqueous phase or into another lipid phase, or in both. They can also be applied to the sheet prior to the introduction of the lipid and aqueous phase. They can even be introduced during the manufacturing process of the sheet itself.

Release of the lipid phase from the beads or capsules can be the result of the rupture of the coating or from the matrix. This may be the result of physical factors such as pressure, strain or by shearing forces upon use of the sheet product, e.g. by rubbing the product to the skin or to a surface. Release of the lipid phase may be due to the semi-permeable or porous nature of the bead or its coating or due to external factors such as contact with liquid media that cause the lipid phase to become extracted, or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact with the aqueous phase when using the sheet product, cause the capsules to disintegrate.

The beads or capsules can be made according to methodologies generally known in the art, for example by emulsion polymerisation.

The beads or capsules may be applied to any portion of the sheet but preferably they are concentrated at the surface or in the upper surface portion of the sheet. This allows maximal transfer of the lipid phase to the skin or to the surface to which the product is applied.

The beads or capsules can be applied to the sheet in dry form by dusting, sifting, spraying and the like methods. They can also be printed or roll-coated in the form of a suitable liquid or paste. They can also be mixed with a suitable liquid, which can be a solvent that is inert towards the beads, or water, or the aqueous phase, and sprayed onto the sheet.

Embodiments of the present invention are those wherein the lipid phase has the composition as described under I or II hereinafter.

In a preferred embodiment, the composition of the lipid phase will have a melting point or range of above 25 °C, preferably in the range of 30 to 45 °C, more preferably in the range of 32 to 40 °C.

The water content of the preferred compositions of the lipid phase is low, e.g. lower than 10 %, preferably lower than 6 %, more preferably lower than 3 %. In particular, the preferred compositions will be water free.

In a preferred embodiment I of the present invention, the lipid phase contains one or more fatty acid mono-, di- or triglycerides, or natural oils comprising mono-, di- or triglycerides as well as the hydrogenated derivatives of said natural oils. The fatty acids in said glycerides may be synthetic or derived from natural oils, including hydrogenated derivatives thereof. The fatty acids contain from 12 to 24, preferably from 16 to 20 carbon atoms.

A particular example of a hydrogenated derivative of a natural oil is hydrogenated castor oil.

### Embodiment I

In embodiment I, the lipid phase comprises one or more mono-, di- or triglycerides, in particular a C₁₂₋₂₄ fatty acid mono-, di- or triglyceride, or more in particular a C₁₆₋₂₀ fatty acid mono-, di- or triglyceride. In a particularly preferred embodiment I, the lipid phase comprises one or more triglycerides, in particular C₁₂₋₂₄ fatty acid triglycerides, or more in particular C₁₆₋₂₀ fatty acid triglycerides. Particular examples of such triglycerides are glyceryl stearate, glyceryl oleate, glyceryl laurate, glyceryl myristate, cocoglycerides, hydrogenated palm glycerides.

The total amount of mono-, di- or triglyceride(s) in the lipid phase of the preferred embodiments I in particular is at least 50 %, preferably at least 70 %, more preferably at least 90 %, w/w of the total amount of components making up the lipid phase. More preferably, the total amount of triglyceride(s) in the lipid phase of the preferred embodiments I is at least 50 %, more preferably at least 70 %, still more preferably at least 90 %, w/w of the total amount of components making up the lipid phase.

### Embodiment II

In a preferred embodiment II, the lipid phase contains C₁₂-C₅₀-fatty alcohols, in particular the C₁₂-C₂₄-fatty alcohols, that are derived from natural fats, oils or waxes such as, for example, myristyl alcohol, 1-pentadecanol, cetyl alcohol, 1-heptadecanol, stearyl alcohol, lauryl alcohol, oleyl alcohol, palmityl alcohol, cetearyl alcohol, 1-nonadecanol, arachidyl alcohol, 1-heneicosanol, behenyl alcohol, brassidyl alcohol, lignoceryl alcohol, ceryl alcohol or myricyl alcohol as well as Guerbet alcohols.

Of particular interest for use in the invention are C₁₄-C₁₈-fatty alcohols as well as C₁₆-C₁₈-Guerbet alcohols.

The total amount of one or more of the C₁₂-C₅₀-fatty alcohols present in the lipid phase, relative to the total weight amount of the lipid phase, is in the range of 1 - 30 % (w/w), preferably of 1 - 20 % (w/w) more preferably from 1 -10 % (w/w).

### Application of the lipid phase

The lipid phase may be applied to the sheet in various ways. Preferably the lipid phase is applied at the surface or at the surface portion of the sheet, on one or on both sides.

The lipid phase can be applied evenly or non-evenly to the sheet, non-evenly meaning that the distribution of the amount of the lipid phase varies over the area of the sheet, i.e. some areas of the sheet can have greater or lesser amounts of the lipid phase. Preferably the lipid phase is evenly applied to the area of the sheet.

The lipid phase can be applied discontinuously or continuously to one or both sides of the fabric, or it may even be applied as a complete covering of one or both surfaces of the fabric.

The lipid phase preferably is applied in a discontinuous pattern, to one or both sides of the sheet. To this purpose the lipid phase is applied in a predetermined, controlled manner to specific areas of the sheet. A discontinuous pattern is one in which the lipid phase has been applied to distinct regions separated by regions of the sheet which are free of the lipid phase. The lipid phase in that instance is applied to defined parts or regions of the sheet which may take a variety of forms. The lipid phase may in particular be applied as described above more generally for the application of both phases. Particular forms in which the lipid phase may be applied are, e.g. stripes, dots or spots, geometric configurations, either of regular or irregular shape, for example circles, ellipses, squares, rectangles and the like, logos, text, letters or any other noncontinuous pattern, including the patterns described hereinabove more generally for the application of the lipid and aqueous phase.

Discontinuous patterns also comprise essentially networks of larger patterns of the lipid phase. In a preferred embodiment, the lipid phase is present as discrete stripes which can be disposed discontinuously, i.e. interrupted, or preferably continuous over the whole surface of the wipe. The stripes may also form a pattern of discrete segments which collectively comprise a stripe or they may have a repetitive pattern such as a sinusoidal shape or wave-like and the like pattern. If waving stripes are selected, preferably the stripes are in phase, so that parallelism is maintained and each stripe remains equally spaced from the adjacent stripes.

The stripes are preferably oriented in the machine direction, for ease of manufacture.

In another execution more than one lipid phase may be applied to one or both sides of the sheet. For example one lipid phase may be applied on the entire surface or part of the surface of one side of the sheet, whereas another lipid phase is applied on the entire other side or only partly, either with the same or another pattern than the other lipid phase. In particular such embodiments are those having two different lipid phases on the same side e.g. in parallel stripes or other patterns with the same or different colors.

In a particular embodiment, not more than half of the surface of the sheet, either on one side or, which is preferred, on both sides is carrying or covered by the lipid phase. In a preferred embodiment, the lipid phase is present at the surface on both sides, covering not more than 50 % of the sheet's surface, in particular covering not more than 35 % or not more than 25 % of the surface. In a particularly preferred embodiment, the lipid phase is present as stripes, in particular as parallel stripes running in parallel with the side of the sheet, covering not more than half or, more in particular 25 % of the surface. In another particularly preferred embodiment, the lipid phase is present as dots, equally spread over the entire surface of the sheet, covering not more than 50 % of the surface.

There can be embodiments with more or less regularly shaped dots, other embodiments have circle-shaped dots, others have ellipsoids, while still others have mixed patterns, e.g. combinations of circles and ellipsoids, of regularly shaped dots and circles and the like.

In case of stripes, the width thereof preferably is between 1 to 10 mm, more preferably between 3 to 7 mm. In case of dots, round shapes are preferred, e.g. circles or ellipsoids, with an average diameter between 1 to 10 mm, more preferably between 3 to 7 mm. There can be stripes with different widths on one product, and there can be dots of different size on one product. An example of an embodiment of the latter is a sheet with circles of a certain size and ellipses of a different size, or of circles with different sizes.

The lipid phase may be colorless or colored, i.e. mono- or multi-colored. Multi-colored patterns are obtained by applying several lipid phases that have been dyed differently. A colored lipid phase will alert the user of the fact that the sheet is covered by a special material that contains an active ingredient or it may also make the product aesthetically attractive.

In another embodiment the sheet itself is colored, either at both sides or at one side, over the complete surface or only at parts. If the color is present only at parts of the sheet it preferably will take the shapes and forms described in connection with the patterns that the lipid phase may take. In another embodiment only the space between the surface portions at which the lipid phase is applied is colored thus leaving the areas of the lipid phase uncolored. In this way, the patterns of the lipid phase will appear as uncolored patterns.

A preferred pattern for coloring the sheet is in stripes, in particular stripes oriented in the machine direction. Examples of such embodiments are those wherein the colored stripes or the area between the colored stripes are covered with lipid phase. In the former instance the lipid phase stripes are colored, in the latter they are uncolored.

The lipid phase, which itself can be colored or uncolored, may be applied to the colored sheet in a number of different ways.

In case of sheets having a completely colored surface, the lipid phase can be applied over the whole surface thus resulting in a different or altered color, e.g. a more pale color where the lipid phase is white or opaque. The lipid phase can also be applied in certain patterns, thus resulting in multicolored products or where the lipid phase is white or opaque in products with mono-colored patterns. Also in this instance, the preferred pattern is in stripes.

In still a further embodiment, the sheet is colored in certain patterns and the lipid phase is applied on these patterns or part of these patterns. Also in this instance the lipid phase may be colored or uncolored, i.e. white, opaque or transparent. In case the lipid phase is white or opaque its thickness may be selected such that the color of the underlying section of the sheet is visible thus giving the consumer the impression that a lipid phase containing a particular ingredient is present.

The lipid phase is typically applied in an amount of about 3 to 40 g/m², preferably from about 10 to about 20 g/m², either on one side or, preferably, on both sides of the sheet. Or, alternatively, the lipid phase is applied in an amount of about 0.06 g to 0.8 g per gram of substrate, preferably from about 0.20 g to 0.40 g per gram of dry substrate.

The lipid phase can be applied to the sheet by any method that can be used to contact or impregnate a liquid or molten lipid material to or in a sheet. The lipid phase may be applied by bathing the sheet into liquid lipid phase. Where the latter is solid or semi-solid at room temperature, it is liquefied by melting or dissolving into a suitable solvent which is evaporated afterwards.

The lipid phase can also be applied by any method that allows coating of the lipid material onto the surface of the sheet. As used herein the term 'coating' refers to printing, covering, overlaying, finishing, spraying, extruding, laminating or any other method of applying the phase to the surface of the sheet.

A particular coating technique is extrusion wherein the composition is forced through tubes in contact with the sheet while the sheet passes across the tube. A preferred technique comprises contacting the sheets with a heated head equipped with a slit blade, i.e. a blade having cut-out areas, wherefrom the lipid phase, in molten state, is extruded. Another preferred coating technique involves the so-called hot melt process which comprises spraying the liquefied lipid phase from a heated spraying head or nozzle. Another application technique involves spraying or drippling the composition on a rotating surface such as calender roll that then transfers the composition to the surface of the substrate.

Still another technique is based on traditional printing technologies which comprise, for example, screen printing, roller printing and gravure printing. In general, printing comprises techniques wherein a rotating surface is provided with elevations (by engraving, embossing or similar techniques) and the elevations are contacted with the liquefied lipid phase, e.g. by running it through a bath with liquefied phase one, and thus printed on the sheet. Another technique to apply the lipid phase is by using a screen printing procedure where the molten lipid phase is introduced into a rotating roll and squeezed through a metal screen which covers the roll. This leads, depending on the design of the screen, to a defined pattern on the fabric like stripes, dots, squares, circles and the like, or even logos and text.

A further technique to apply the lipid phase onto the sheet is by roller-ball application which comprises contacting a ball which is in direct contact with the sheet, with lipid phase in liquid state and transferring it through a rolling movement onto the sheet. Depending on the desired pattern of the lipid phase on the sheet, there can be several of such roller-ball applicators mounted next to one another, or after one another. They may contain the same or different lipid phases.

The lipid phase may be applied by high-pressure coating. In one embodiment of this procedure the lipid phase is applied via extrusion through appropriate nozzles, under high pressure. Specially shaped nozzles may be used resulting in particular patterns. For example there can be nozzles that result in circles, stars, squares, or other geometric shapes or even irregularly shaped patterns.

The lipid phase may also be applied by a combination of these application techniques.

The lipid phase may also be applied to the sheet in dry form as particles or as powder. In one type of embodiments the lipid phase is applied as beads or small capsules, e.g. by drippling or screen printing. After application the particles are caused to melt thereby forming small dots in or on the sheet.

The lipid phase preferably is applied in liquid form, e.g. in its molten form.

The lipid phase can be applied either to one surface of the sheet or both surfaces, preferably to both surfaces.

In the instance of multi-layered sheet products, the lipid phase may be applied on one or more of the sheet layers. This equally applies to the aqueous phase. Embodiments of multi-layered sheets are three-layered sheets wherein the outer layers have the same or different lipid phases and the inner layers have an aqueous phase or any other combination.

The lipid phase may be applied in liquid form while being in admixture with water, which can be colored or uncolored and which is removed after application to result in a dry or essentially dry product. 'In liquid form' in this context means that the lipid phase is liquid in itself or is liquefied by heating, e.g. by heating in the water in which it is applied. The lipid phase is kept liquid all along the process. In the instance of a solid lipid phase, it is only allowed to solidify after removal of the water that has been added. In one embodiment, the lipid phase is mixed with hot water whereupon the lipid/water mixture is applied to the sheet. The water is subsequently evaporated which may be accomplished by a variety of means, e.g. by simply allowing the water to evaporate, by passing the sheet over one or more heated rolls, thus forcing the water to evaporate, by applying dry air, either heated or not, by applying reduced pressure.

In the execution where the water is colored, it will diffuse into the sheet and after its evaporation leave the sheet colored. The lipid phase that has been applied in this execution may be uncolored, in which case it will appear as white or lighter areas. Or the lipid phase may be colored which will result in a multi-colored product. In another execution, the lipid phase in this process is colored and uncolored water is used resulting in products wherein the lipid phase areas are colored and the areas and the other areas are uncolored. The thus obtained products may subsequently be treated with aqueous phase which may be colored or not resulting in products with even more color combinations.

In one type of embodiments, the lipid phase is applied as a layer on the sheet, either continuously or discontinuously, at one or both sides of the sheet and this layer is dotted with particles of lipid phase material that are punched into the surface of the lipid layer by application of pressure. The material of the dots may be the same or different as that of the lipid layer.

The lipid phase preferably is applied in such manner that it will remain on the fabric surface during the manufacturing process and storage. This can be conveniently accomplished by applying the lipid phase above its melting temperature, e.g. by spraying or coating it when molten to the surface of the sheet and subsequently allowing it to cool below its melting point so that the phase solidifies.

The lipid phase preferably is applied such that it is present at the surface of the sheet because of its physical location in that instance, the lipid phase is readily available to be spread onto the skin during usage. As a result, the effectiveness with which the lipid phase is transferred to the skin during use, the availability and therefore the effectiveness of active ingredients is increased compared to products where the active agent is simply incorporated into a single continuously applied phase.

In preferred embodiments, the melting point or range of the lipid phase is above 25 °C, or within the temperature ranges specified above, because this allows to apply the lipid phase in liquid (molten) state to the sheet, and subsequently, after it having been cooled, to be present in solid state on the sheet. Additionally this allows a more convenient and easy after-treatment of the sheet to which the lipid phase has been applied in this manner, with the aqueous phase. This allows the two phases to be applied in such manner that they do not mix or interact. In preferred embodiments, the lipid phase is applied such that it forms a weak non-brittle film on the sheet. Sheets that have been treated this way are particularly stable, in particular during storing, essentially because mixing of the two phases is avoided. Additionally such sheets will allow the lipid phase to melt upon contact with the skin, thus allowing a local mixing or emulsification of both phases.

### The aqueous phase

The aqueous phase can be any of the art-known aqueous based formulations used to impregnate wipes. Beside water the aqueous phase may also contain further ingredients or additives such as surfactants, emulsifiers, consistency factors, conditioners, moisturizers, thickeners, preservatives, active ingredients, in particular dermatologically active ingredients, fragrances and the like. Active ingredients as mentioned herein comprise, for example, anti-inflammatories, anti-bacterials, antifungals and the like agents. Active ingredients suited for topical applications are particularly preferred.

The aqueous phase may contain suitable dyes which preferably are hydrophylic. In one type of embodiments, the lipid phase is applied discontinuously as a layer e.g. in the form of stripes leaving areas with only aqueous phase, which areas are colored. This allows the manufacture of sheet products with colored patterns, e.g. colored lines or even multicolored patterns when the lipid phase itself is also colored.

The aqueous phase may further contain lipophilic dyes, which upon contact with the lipid phase migrate into that phase and cause it to become colored.

The aqueous phase may further contain one or more preservatives such as, for example, phenoxyethanol, C₁-₄ alkylparabens and their salts, in particular their alkali metal salts such as sodium salts (e.g. C₁-₆ alkyl parabens such as methyl, ethyl, propyl, isopropyl, butyl paraben and the like parabens), chlorohexidine, formaldehyde or formaldehyde releaser, benzyl alcohol, chloroxylenol, phenoxyethanol, methylchloroisothiazolinone, methylisothiazolinone, sodium benzoate, chlorohexidine digluconate methyldibromo glutaronitrile, sodium borate, 5-bromo-5-nitro-1,3-dioxane, alcohol, benzoic acid, dehydroacetic acid, diazolidinyl urea, dichlorobenzyl alcohol, glucose oxidease, hexamidine diisethionate, imidazolidinyl urea, iodopropynyl butylcarbamate, isobutylparaben, isopropylparaben, lactoperoxidease, magnesium nitrate, PEG-4 laurate, phenethyl alcohol, polyaminopropyl biguanide, potassium sorbate, propylene glycol, pyridoxine HCl, quaternium-15, sorbic acid, triclosan, tocopherol and the like.

Suitable surfactants for the aqueous phase comprise:
alkyl sulfates, e.g. sodium lauryl sulfate, ammonium lauryl sulfate, sodium cetearyl sulfate;
alkyl sulfoacetates, e.g. sodium lauryl sulfoacetate;
alkyl ether sulfates e.g. sodium laureth sulfate, sodium trideceth sulfate, sodium oleth sulfate, ammonium laureth sulfate;
alkyl ether sulfosuccinates, e.g. disodium laureth sulfosuccinate;
alkyl glycosides, e.g. decyl glucoside, lauryl glucoside;
alkyl isothionates;
amphoterics, e.g. cocamidopropyl betaine, sodium cocoamphoacetate, sodium lauroamphoacetate, disodium lauroamphodiacetate, disodium cocoamphodiacetate, sodium lauroamphopropionate, disodium lauroamphodipropionate, potassium or ammonium salts of the aforementioned amphoterics, capryl/capramidopropyl betaine, undecyleneamidopropyl betaine, lauramidopropyl betaine and fatty alcohol polyglycol ethers.

Suitable conditioners are e.g. alkylamido ammonium lactate, cetrimonium chloride and distearoylethyl hydroxyethylmonium methosulfate and cetearyl alcohol, cetyl dimethicone, cetyl ricinoleate, dimethicone, laureth-23, laureth-4, polydecene, retinyl palmitate, agents selected from glyceryl monooleate and cocoglucoside including mixtures thereof (in particular the product 'Lamesoft^{®}' of Cognis which is a mixture of these two components), quaternized protein hydrolysates, quaternized cellulose and starch derivatives, quaternized copolymers of acrylic or methacrylic acid or salts, quaternized silicone derivatives, silicone oils, cyclomethicones, and the like agents, including mixtures thereof.

Suitable thickeners are e.g. acrylates/steareth-20 methacrylate copolymer, carbomer, carboxymethyl starch, cera alba, dimethicone/vinyl dimethicone crosspolymer, propylene glycol alginate, hydroxyethylcellulose, hydroxypropyl methylcellulose, silica, silica dimethyl silylate, xanthan gum, hydrogenated butylene/ethylene/styrene copolymer.

The aqueous phase may further comprise film-forming substances like chitosan and derivatives thereof, derivatives of poly acrylic acid, polyvinyl pyrrolidone and its derivatives, and the like.

The aqueous phase may contain pH sensitive components, i.e. components that change properties upon change of pH. The change of pH may occur when contacting the sheet product with the skin whereupon the pH changes from the pH of the product which usually is about pH 7 to the skin pH which is about pH 5.5. pH sensitive agents for example comprise particular emulsifiers, stabilizers, surfactants viscosity regulating agents, chelators and the like.

In one embodiment an appropriate emulsifier is selected that is pH sensitive in this pH range in that it changes its emulsifying capacity, preferably increases its emulsifying capacity, so that upon contact with the skin an emulsification process occurs causing an interaction between the aqueous and lipid phases.

The aforementioned change of pH that occurs upon application of the product to the skin may also promote the release from active ingredients, in particular actives that are pH sensitive, e.g. actives having a pH dependent solubility.

### Application of the aqueous phase

The aqueous phase may be applied to the sheet using methods generally known in the art for applying aqueous liquid lotions such as spraying, dripping, immersing and the like techniques. A preferred application method for the aqueous phase is by spraying with a suitable nozzle or by drippling, for example by using a perforated tube having holes or slits. The immersing technique can be done by running the sheets through a bath holding the aqueous phase and subsequently controlling the amount of liquid that is absorbed by pressing.

The aqueous phase may be applied in various ways as described for the lipid phase, evenly or non-evenly, continuously or non continuously, at the surface or surface portion or, preferably, throughout the whole of the sheet material. Optionally some parts of the sheet can be left dry, i.e. not having the lipid and the aqueous phase, or some parts can only have the lipid or the aqueous phase. The aqueous phase may be applied at both sides or only at one side of the sheet.

The aqueous phase can be applied in various amounts, for example in an amount from about 0.1 g to about 10 g per gram of substrate and is typically applied in an amount from about 1.0 g to about 10 g per gram of substrate, preferably from about 2.0 g to about 5 g per gram of substrate, most preferably from about 2 g to about 4.5 g per gram of dry substrate, most preferably about 3.7 to about 3.8 g per gram substrate. Or, the aqueous phase is applied in an amount of about 4 to about 8 g per wipe sized 17.2 x 21 cm, most preferably about 6 g per wipe.

It may also be advantageous to only apply the aqueous phase to only those areas (or that side) of the sheet which have (or has) not already been covered with the lipid phase.

Since in many cases the product is used as a cleansing article it is useful to design the aqueous phase as cleanser. Soils that are most difficult to clean are either water insoluble and/or strongly adhere to the skin. Therefore the aqueous phase is formulated such that it is capable of taking up water-insoluble materials.

### Further Phases

In another embodiment of the invention a third layer is applied to the sheet, which is made of polymeric material, hereafter referred to as polymeric layer. One or more polymeric layers may be applied to the sheet. The term 'polymeric layer', whenever used hereinafter refers to one or more polymeric layers.

The polymeric layer may be applied to one side of the sheet or to both sides.

The polymeric layer is made of a suitable polymer such as polyethylene, polypropylene, polyester, a silicone and the like, including mixtures thereof. The polymeric layer may contain other materials, such as fillers or dyes. In the latter instance the area of the sheet covered with the polymeric layer will occur as colored areas. In case several polymeric layers are applied, layers with different colors may be used thus resulting in different colored patterns.

The polymeric layer may be applied to the sheet similarly as described for the application of the lipid phase. For example, it may be applied continuously, i.e. over the whole surface of the sheet, or discontinuously, e.g. in patterns, e.g. as stripes, spots or other figures. In the instance where the polymeric layer does not cover the whole surface, the lipid phase may cover both the areas of the sheet that are covered by the polymeric layer and the other areas.

The lipid layer may be applied onto the polymeric layer thus forming a double layer. The polymeric layer needs not be completely covered by the lipid phase, i.e. some parts may remain uncovered.

The polymeric layer may also be applied to the areas that are not covered by the lipid phase. For example the lipid phase may be applied as a layer in a discontinuous fashion and the polymeric phase is applied at the spots without lipid phase. In one particular embodiment the lipid phase is applied as stripes and the polymeric layer is put in the area between these stripes thus forming a pattern of alternating stripes of lipid phase and polymeric layer. This may for example be done at one side of the sheet while the aqueous phase is put at the other side.

The polymeric layer may be semi-solid so that it can be disrupted upon application of a product having such a layer. Semi-solid polymeric layers are made of polymers that have a waxy, creamy or similar constituency. In that instance the polymeric layer can also be applied as an external coating onto the sheet, covering one or both sides, covering parts or the whole surface. It may also cover parts or the whole of the lipid layer.

The lipid phase that covers the polymeric layer may be colored or uncolored. In the former instance, the polymeric layer preferably is uncolored or white although it may be colored also. In the instance where the lipid phase is uncolored, the polymeric phase preferably is colored, although it may also be white or uncolored.

The polymeric phase may be applied for improving or promoting the transfer of the lipid phase that is coated thereon to the user's skin. Using a colored polymeric layer, or a colored lipid phase, or both, results in an appearance, disappearance or respectively change of color when the sheet product is used and the lipid phase is transferred to the skin.

The polymeric layer is applied to the sheet using art-known methods to coat sheet like materials with a polymeric layer. For example the polymeric layer can be applied by screen printing, gravure printing, roller printing, embossing, spraying, drippling, bathing and the like techniques.

In some embodiments of this invention the products may contain two or more lipid phases with different stability towards the aqueous phase. This allows one phase to interact more quickly with the aqueous phase than the other. This may find application in products where a gradual of active ingredient is desired or the release of a sequence of two or more active ingredients.

### Additional ingredients for either one or both phases

The lipid and/or the aqueous phase may contain further ingredients that may be present in one or in both phases.

### Active ingredients

The lipid and/or the aqueous phase further may contain active ingredients for application to the skin. The lipid phase preferably contains oil-soluble or hydrophobic active agents, while the aqueous phase preferably contains water-soluble or hydrophilic active agents. However by using suitable emulsifiers oil-soluble or lipophilic active ingredients can be incorporated into the aqueous phase and vice versa, water-soluble or hydrophilic agents can be incorporated in the lipid phase.

Products having a lipid and/or an aqueous phase that contains one or more active ingredients constitute particularly attractive embodiments of the present invention. Particularly preferred embodiments are those wherein the active ingredients are present in the lipid phase.

The active ingredients can be present in particular combinations.

The active ingredients, which may be lipophilic or hydrophilic, can be mixed with or incorporated into suitable carriers. These comprise any skin-acceptable inert materials that are known for formulating active ingredients. The carriers can be finely or more coarsely divided powders, or even granulates. They can comprise starches, sugars, binders, lubricants, diluents, fillers, disintegrating agents, granulating agents and the like components. The nature of the carrier materials will depend on the active ingredient that is formulated therein and on the type of formulation that is desired.

Particular carriers for incorporating active ingredients are beads wherein the active ingredient is entrapped in some form. The terms 'beads' or 'polymeric beads' are meant to comprise any form of discrete, free-flowing powders, beads or capsules which envelope, coat or contain an active ingredient in a mono- or polymeric matrix or capsule. These terms are also meant to include porous beads or 'microsponges' and 'microcapsules', the latter being beads of smaller size. The beads may be coated with a suitable coating material that protects the interior of the bead or controls the release of the active ingredient entrapped therein. The coating on the bead itself may contain the active ingredient in which case the coating is layed on an inert core.

Formulating an active ingredient in beads can be for protecting the active from environmental factors but is mostly done for allowing controlled release of the active.

A particular type of beads are small beads or capsules, having an average diameter which is in the micrometer range, although the average diameter can be as small as even 200 nm.

This type of capsules can be liposome-based, made for example of phospholipids such as lecithin, phosphatidyl ethanolamine, phosphatidyl serine, phosphatidic acid and the like. This type of capsules also can be made of starch, cellulose, porous gelatin and the like.

The capsules or beads can also be relatively larger, having average sizes in the mm or 0.1 mm range. This type of capsules or beads can be made of materials such as agar, glycolic acid polymers, and further components such as water, mineral oils, glycerin. They may contain further ingredients such as preservatives, dye(s), and the like.

Another type of beads or microcapsules are microsponges. These are materials sized from about 5 to about 300 µm (average diameter) having a large inner surface. These are obtained by polymerization of particular monomers. An active ingredient can be entrapped therein either during this polymerization process or afterwards.
Microsponge-based carriers may be used to protect the active ingredient entrapped therein or for controlled release purposes.

The capsules may optionally contain one or more suitable disintegrating agents, in particular those mentioned in this specification. Upon contact with the appropriate external factor, the disintegrating agents will cause the capsules to break open thus allowing release of the active ingredient entrapped therein.

The capsules can be incorporated into the lipid or the aqueous phase or into both. They can also be applied to the sheet prior to the introduction of the lipid and aqueous phase. They can even be introduced during the manufacturing process of the sheet itself.

Release of the active from the beads or capsules can be the result of the rupture of the coating or the matrix. This may be the result of physical factors such as pressure, strain or by shearing forces upon use of the sheet product, e.g. by rubbing the product to the skin or to a surface. Release of the active ingredient may be due to the semi-permeable or porous nature of the bead or its coating or due to external factors such as contact with liquid media that cause the active ingredient to become extracted, or to dissolve or disintegrate the bead or its coating, or by temperature effects. The capsules can also be disintegrated under influence of certain chemicals, in particular by disintegrating agents incorporated into the capsules. Particular embodiments of the latter are capsules containing suitable amounts of bicarbonate and an organic acid which, upon contact with water, e.g. upon contact with the aqueous phase when using the sheet product, cause the capsules to disintegrate.

The beads or capsules can be made according to methodologies generally known in the art, for example by emulsion polymerisation.

The beads or capsules may be applied to any portion of the sheet but preferably they are concentrated at the surface or in the upper surface portion of the sheet. This allows maximal transfer of the active ingredient to the skin or to the surface to which the product is applied.

The beads or capsules can be applied to the sheet in dry form by dusting, sifting, spraying and the like methods. They can also be printed or roll-coated in the form of a suitable liquid or paste. They can also be mixed with a suitable liquid, which can be a solvent that is inert towards the beads, or water, or the aqueous phase, and sprayed onto the sheet.

Examples of active agents which may be hydrophobic or hydrophilic for use in the products of the invention comprise anti-microbials, e.g. anti-bacterials and antifungals, anti-inflammatory agents, anti-irritating compounds, anti-itching agents, moisturising agents, skin caring ingredients, plant extracts, vitamins, and the like. Examples of such ingredients comprise complexes of PVP and hydrogen peroxide, anti-inflammatories as, plant extracts, bisabolol, panthenol, tocopherol, actives for anti-stinging, anti-irritants, anti-dandruffs, for anti-ageing e.g. retinol, melibiose etc. Other suitable actives are e.g. Medicago officinalis, Actinidia chinensis, allantoin, Aloe barbadensis, Anona cherimolia, Anthemis nobilis, Arachis hypogaea, Arnica montana, Avena sativa, beta-carotene, bisabolol, Borago officinalis, butylene glycol, Calendula officinalis, Camellia sinensis, camphor, Candida bombicola, capryloyl glycine, Carica papaya, Centaurea cyanus, cetylpyridinium chloride, Chamomilla recutita, Chenopodium quinoa, Chinchona succirubra, Chondrus crispus, Citrus aurantium dulcis, Citrus grandis, Citrus limonum, Cocos nucifera, Coffea arabica, Crataegus monogina, Cucumis melo, dichlorophenyl imidazoldioxolan, Enteromorpha compressa, Equisetum arvense, ethoxydiglycol, ethyl panthenol, farnesol, ferulic acid, Fragaria chiloensis, Gentiana lutea, Ginkgo biloba, glycerin, glyceryl laurate, Glycyrrhiza glabra, Hamamelis virginiana, heliotropine, hydrogenated palm glycerides, citrates, hydrolyzed castor oil, hydrolyzed wheat protein, Hypericum perforatum, Iris florentina, Juniperus communis, lactis proteinum, lactose, Lawsonia inermis, linalool, Linum usitatissimum, lysine, Magnesium aspartate, magnifera indica, Malva sylvestris, mannitol, mel, Melaleuca alternifolia, Mentha piperita, menthol, menthyl lactate, Mimosa tenuiflora, Nymphaea alba, olaflur, Oryza sativa, panthenol, paraffinum liquidum, PEG-20M, PEG-26 jojoba acid, PEG-26 jojoba alcohol, PEG-35 castor oil, PEG-40 hydrogenated castor oil, PEG-60 hydrogenated castor oil, PEG-8 caprylic/capric acid, Persea gratissima, petrolatum, potassium aspartate, potassium sorbate, propylene glycol, Prunus amygdalus dulcis, prunus armeniaca, Prunus persica, retinyl palmitate, Ricinus communis, Rosa canina, Rosmarinus officinalis, rubus idaeus, salicylic acid, Sambucus nigra, sarcosine, Serenoa serrulata, Simmondsia chinensis, sodium carboxymethyl betaglucan, sodium cocoyl amino acids, sodium hyaluronate, sodium palmitoyl proline, stearoxytrimethylsilane, stearyl alcohol, sulfurized TEA-ricinoleate, talcum, thymus vulgaris, Tilia cordata, tocopherol, tocopheryl acetate, trideceth-9, Triticum vulgare, tyrosine, undecylenoyl glycine, urea, Vaccinium myrtillus, valine, zinc oxide, zinc sulfate and the like.

Of particular interesst are active ingredients, that can be used for treating skin that shows inflammatory reactions, that is irritated, red or damaged. Examples of such agents are zinc compounds or sulphur.

Further active ingredients that can be used are known under the tradename Generol™.These comprise ethoxylated and non-ethoxylated phytosterines.

The active ingredients can be present, depending on the nature of the ingredients and their application, in various concentrations, but usually are present in a quantity in the range of 0,01 - 10 % (w/w), preferably from 0,1 - 7 % (w/w) and more preferably 1 - 5 % (w/w), w/w expressed to the total weight of the lipid or to the aqueous phase.

Typical examples of anti-microbial agents are those active against gram-positive bacteria such as 2,4,4'-trichloro-2'-hydroxydiphenyl ether, chlorohexidine (1,6-di-(4-chlorophenyl-biguanido)hexan) or TCC (3,4,4'-trichlorocarbanilide). Furthermore many odorants and etheric oils have anti-microbial activity. Typical examples are the active ingredients eugenol, menthol and thymol in clove, mint and thyme oil. Further interesting natural deodorizing agents having anti-microbial properties are the terpene alcohol farnesol (3,7,11-trimethyl-2,6,10-dodecatrien-1-ol) and chitosan. Also glycerine monolaurate, glycerine stearate, glycerine oleate as well as glycerine dioleate have been found to possess anti-microbial activity and are particularly attractive for use in products that are applied on babies because of their mildness and lack of side effects. The quantity of anti-microbial agents can vary but usually is in the range of about 0.1 to 2 % (w/w) - relative to the total amount of the lipid and/or the aqueous phase

Biogenic active ingredients are for example tocopherol, tocopherol acetate, tocopherol palmitate, ascorbic acid, desoxyribonucleinic acid, retinol, bisabolol, allantoin, phytantriol, panthenol, α-hydroxycarbonic acids, amino acids, ceramides, pseudoceramides, essential oils, extracts and vitamine complexes.

Glycerine esters can be used in larger quantities (see above).

### Moisturizers

The lipid and/or aqueous phase can further contain one or more moisturizers. These are added to improve the sensoric properties as well as to regulate skin hydratation. These agents additionally can improve the penetration of the composition in or into the sheet.

Moisturizers typically are present in quantities of 1 -20 % (w/w), preferably of 5 - 15 % (w/w), and more preferably 5 -10 % (w/w) relative to the total amount of the lipid and/or the aqueous phase.

Suitable moisturizers are a.o. amino acids, pyrrolidone carbonic acid, lactic acid and its salts, lactitol, urea and urea derivatives , ureic acid, glucosamine, creatinine, hydrolysis products of collagen, chitosan or chitosan salts/-derivatives , and in particular polyols and polyol derivatives (e.g. ethylene glycol, propylene glycol, butylene glycol, pentylene glycol, hexylene glycol, erythrite, 1,2,6-hexanetriol, polyethylene glycols such as PEG-4, PEG-6, PEG-7, PEG-8, PEG-9, PEG-10, PEG-12, PEG-14, PEG-16, PEG-18, PEG-20, PEG-135, PEG 150), sugar and sugar derivatives (a.o. fructose, glucose, maltose, maltitol, mannite, inosite, sorbite, sorbityl silandiol, sucrose, trehalose, xylose, xylit, glucuronic acid and its salts), ethoxylated sorbitol (Sorbeth-6, Sorbeth-20, Sorbeth-30, Sorbeth-40), honey and hydrogenated honey, hydrogenated starch hydrolysates, as well as mixtures of hydrogenated wheat protein, hydrolyzed milk protein, lecithin, pythantriol, hyaluronic acid and salts thereof, and PEG-20-acetate copolymers. Particularly preferred moisturizers are glycerine, diglycerine and triglycerine.

The products according to this invention can be used as anti-perspirants or deodorants, in particular as wipes or tissues for use in these applications. In products for these applications either one or both phases contain actives that have deodorizing and /or anti-perspirant properties. Actives that can be used to this purpose are anti-perspirant agents such as, for example, aluminium chlorohydrates, aluminium-zirconium-chlorohydrate as well as zinc salts. Other such agents comprise aluminium hydroxylactates as well as acid aluminium/zirkonium salts. A particularly suitable chlorohydrate is the compound of formula [Al₂(OH)₅Cl]·2.5 H₂O. Further such agents are aluminium-zirconium-tetrachlorohydroxy-glycine-complexes. Esterase inhibitors can be added as further deodorizing agents, i.e. agents such as trialkyl citrates such as trimethylcitrates, tripropyl citrates, triisopropyl citrates, tributyl citrates and in particular triethyl citrates. Further esterase inhibitors are sterol sulfates or -phosphates, such as, for example, lanosterine-, cholesterine-, campesterine-, stigmasterine- and sitosterine sulfate respectively -phosphate, dicarbonic acids and their esters, such as, for example, glutaric acid, glutaric acid monoethylester, glutaric acid diethylester, adipinic acid, adipinic acid monoethylester, adipinic acid diethylester, malonic acid and malonic acid diethylester, hydroxycarbonic acids and their esters such as, for example, citric acid, malonic acid, tartaric acid or tartaric acid diethylester.

Antibacterial active ingredients that influence the growing conditions and eradicate perspiration decomposing bacteria, or impede their growth, can also be present in the lipid and/or aqueous phase. Examples of such ingredients are chitosan, phenoxyethanol and chlorohexidine gluconate and in particular 5-chloro-2-(2,4-dichlorophenoxy)-phenol.

The products according to the invention can also be used in sunscreen applications and in that instance take the form of sunscreen wipes. In these products the lipid and/or aqueous phase contains one or more sunscreen filters which are for example organic substances that are capable of absorbing ultraviolet radiation and to set free the absorbed energy as longer-wave radiation, e.g. as thermic energy. UVB-filters can be oil or water soluble. As oil-soluble substances there can be mentioned for example:
- 3-Benzylidene campher respectively 3-benzylidene norcampher and derivatives thereof, e.g. 3-(4-methylbenzylidene) campher;
- 4-Aminobenzoic acid derivatives, respectively 4-(dimethylamino)benzoic acid-2-ethylhexyl esters, 4-(dimethylamino)benzoic acid-2-octyl esters and 4-(dimethylamino)benzoic acid amylesters;
- Esters of cinnamonic acid, in particular 4-methoxycinnamonic acid-2-ethylhexylester, 4-methoxycinnamonicacid propylester, 4-methoxycinnamonic acid isoamyl ester, 2-cyano-3,3-phenylcinnamonic acid-2-ethylhexyl ester (octocrylene);
- Esters of salicylic acid, respectively salicylic acid-2-ethylhexylester, salicylic acid-4-isopropylbenzyl ester, salicylic acid homomenthyl ester;
- Derivatives of benzophenones, in particular 2-hydroxy-4-methoxybenzophenone, 2-hydroxy-4-methoxy-4'-methylbenzophenone, 2,2'-dihydroxy-4-methoxybenzophenone;
- Esters of benzalmalonic acid, in particular 4-methoxybenzmalonic acid di-2-ethylhexyl ester;
- Triazine derivatives, such as, for example, 2,4,6-trianilino-(p-carbo-2'-ethyl-1'-hexyloxy)-1,3,5-triazin and octyltriazone;
- Propane-1,3-diones, such as for example, 1-(4-tert.butylphenyl)-3-(4'-methoxyphenyl)propane-1,3-dione;
- Ketotricyclo(5.2.1.0)decane-derivatives.

Water-soluble UV-filter are for example:
- 2-Phenylbenzimidazol-5-sulfonic acid and its alkali-, alkaline earth-, ammonium-, alkylammonium-, alkanolammonium- and glucammonium salts;
- Sulfonic acid derivatives of benzophenones, in particular 2-hydroxy-4-methoxybenzophenone-5-sulfonic acid and its salts;
- Sulfonic acid derivatives of 3-benzylidene campher, e.g. 4-(2-oxo-3-bornylidene methyl)benzol-sulfonic acid and 2-methyl-5-(2-oxo-3-bornylidene)sulfonic acid and its salts.

Typical UV-A-Filters that can be used are derivatives of benzoylmethane, such as, for example, 1-(4'-tert.butylphenyl)-3-(4'-methoxyphenyl)propane-1,3-dione, 4-tert.-butyl-4'-methoxydibenzoylmethane (Parsol 1789), or 1-phenyl-3-(4'-isopropylphenyl)-propane-1,3-dione. Mixtures of UV-A and UV-B-filters can evidently be used too.

Apart from the above mentioned soluble substances, there can also be used insoluble sunscreen pigments, namely finely dispersed metal oxides or metal salts. Examples of appropriate metal oxides are in particular zinc oxide and titanium dioxide as well as oxides of iron, zirconium, silicon, mangan, aluminium and cerium as well as mixtures thereof. Salts that can be used comprise silicates (talcum), barium sulfate or zinc stearate. The particle size of these pigments is sufficiently small, e.g. less than 100 nm, in particular between 5 and 50 nm and more in particular between 15 and 30 nm. The particles can be spherical but can have other shapes too such as ellipsoidal or similar shapes. The surface of the pigments may have been treated, e.g. hydrophilized or made hydrophobic. Typical examples are coated titanium dioxide, e.g. Titanium dioxide T 805 (Degussa) or Eusolex^{®} T 2000 (Merck). Silicones can be used as hydrophobic coating agents, in particular trialkoxyoctyl silanes or simethicones. So-called micro- or nanopigments are particularly attractive for use in sunscreen products.

Apart from both groups of primary light protecting filters that are mentioned above there can also be used secondary light protecting factors. These pertain to the class of anti-oxidants and their activity is based on the interruption or decrease of the photochemical processes caused by solar radiation upon penetration in the skin.

Typical examples of secondary light protecting agents are amino acids such as for example glycine, histidine, tyrosine and tryptophane, including derivatives of amino acids; imidazoles (for example urocanic acid) and derivatives thereof; peptides such as D,L-carnosine, D-carnosine, L-carnosine and derivatives thereof (e.g. anserin). Further agents that can be used are carotinoides, carotenes (for example α-carotene, β-carotene and lycopene) and derivatives thereof; chlorogenic acid and its derivatives; lipoic acid and derivatives thereof (e.g. dihydrolipoic acid), aurothioglucose, propylthiouracil and other thioles (for example thioredoxine, glutathione, cysteine, cystamine and glycosyl-, N-acetyl-, methyl-, ethyl-, propyl-, amyl-, butyl- and lauryl-, palmitoyl-, oleyl-, γ-linoleyl-, cholesteryl- and glyceryl esters) and their salts. Further examples are dilauryl thiodipropionate, distearyl thiopropionate, thiodipropionic acid and derivatives thereof (for example esters, ethers, peptides, lipids, nucleotides, nucleosides and salts), sulfoximine compounds (for example buthionine sulfoximine, homocysteine sulfoximine, butionine sulfone, penta- hexa-, and heptathione sulfoximine). These secondary agents usually are formulated into very low concentrations (e.g. pmol to µmol/kg),

Other secondary agents (usually in small concentrations, as mentioned above) are chelating agents (for example α-hydroxy fatty acids, palmeate acid, phytic acid, lactoferrin), α-hydroxy acids (for example citric acid, lactic acid , malic acid), humic acid, bile acid, bile extracts, bilirubin, biliverdin, EDTA, EGTA and derivatives thereof; unsaturated fatty acids and derivatives thereof such as for example γ-linolenic acid, linoleic acid, oleic acid, folic acid and derivatives thereof, ubiquinones and ubiquinol and derivatives thereof, vitamin C and derivatives thereof (e.g. ascorbyl palmitate, Mg ascorbylphosphate, ascorbyl acetate), tocopherol and derivatives thereof (for example vitamin E acetate), vitamin A and derivatives thereof (e.g. vitamin A palmitate), coniferyl benzoates of benzoic acid, rutinic acid and derivatives thereof, α-glycosyl rutin, ferula acid, furfurylidene glucitol, carnosine, butyl hydroxytoluene, butyl hydroxyanisol, nordihydroguaiac resin acid, nordihydroguaiaret acid, trihydroxybutyrophenone, ureic acid and derivatives thereof, mannose and derivatives thereof, superoxide-dismutase, zinc and derivatives thereof such as zinc oxide, zinc sulphate, selenium and derivatives thereof (e.g. seleno-methionine); stilbene and derivatives thereof (e.g. stilbene oxide, trans stilbene oxide); and any appropriate derivatives of these UV filters.

To improve the rheological behavior there can be added hydrotropes, such as, for example, ethanol, isopropyl alcohol, or polyoles. Polyoles, that can be used in particular have 2 to 15 carbon atoms and at least 2 hydroxyl groups, and optionally have further substituents such as amino or other nitrogen-based substituents. A number of these compounds have been mentioned amongst the moisturizing agents. Typical examples are:
- glycerine;
- alkylene glycoles, such as, for example, ethylene glycol, diethylene glycol, propylene glycol, butylene glycol, hexylene glycol as well as polyethylene glycol with a mean molecular weight from 100 to 1000 Daltons;
- technical oligoglycerine mixtures with a condensation level of 1.5 to 10 such as technical diglycerine mixtures with a diglycerine content of 40 to 50 % (w/w);
- methylol compounds such as in particular trimethylol ethane, trimethylol propane, trimethylol butane, pentaerythrite and dipentaerythrite;
- lower alkyl glucosides, in particular those with 1 to 8 carbon atoms in the alkyl rest, for example methyl- and butyl glucoside;
- sugar alcohols with 5 to 12 carbon atoms, such as, for example, sorbite or mannite,
- sugars with 5 to 12 carbon atoms, such as, for example, glucose or saccharose;
- amino sugars, such as, for example, glucamine;
- dialcohol amines, such as diethanolamine or 2-amino-1,3-propane diol.

As self-tanning agents there can be added dihydroxy acetone.

As perfume oils there can be mentioned mixtures from synthetic or natural odorous substances. Natural odorous substances are extracts from blossoms (lily, lavender, rose, jasmine, neroli, ylang-ylang), from stems and leaves (geranium, patchouli, petitgrain), from fruits (anis, coriander, caraway, juniper), from cortex (bergamot, lemon, orange), from roots (macis, angelic, celery, cardamon, costus, iris, calmus), from wood (pine, sandelwood, guajak, cedar, rose), from herbs and grass (tarragon, lemongrass, sage, thyme), from needles and branches (spruce, fir, pine, mountain pine), from resins and balms (galbanum, elemi, benzoin, myrrh, olibanum, opoponax). Furthermore animal odorous substances can be used, such as, for example, civet and castoreum. Typical synthetic odorous substances are products of the classes of esters, ethers, aldehydes, ketones, alcohols and hydrocarbons.

Etheric oils of lower volatility, which are mostly used as aromatic components, can be added to perform as perfume oils, e.g. sage, chamomile, clove, balm, mint, cinnamon leaf, lime blossom, juniper, vetiver, olibanum, galbanum, labolanum and lavandin. Particular oils are bergamot, dihydro-myrcenol, lilial, lyral, citronellol, phenylethyl alcohol, α-hexylcinnamonic aldehyde, geraniol, benzyl acetone, cyclamene aldehyde, linalool, boisambrene forte, ambroxan, indole, hedione, sandelice, lemon, mandarin, orange, allyl amylglycolate, cyclovertal, lavandin, muskateller sage, β-damascone, geranium bourbon, cyclohexyl salicylate, vertofix coeur, iso-E-super, fixolide NP, evernyl, iraldein gamma, phenyl acetic acid, geranyl acetate, benzyl acetate, rose oxide, romilllat, irotyl and floramate; or mixtures thereof.

The lipid and/or aqueous phase may contain cosmetically acceptable dyes which can be present in quantities in the range of 0,001 to 0,1 % (w/w), relative to the total quantity of the lipid and/or aqueous phase. Oil soluble dyes preferably are used in the lipid phase, water-soluble dyes in the aqueous phase. Preferably, the lipid phase contains one or more dyes, the aqueous phase not. Dyes that can be used in the lipid phase are, for example the C.I. series of oil-soluble dyestuffs, e.g. C.I. 47000, C.I. 67565, C.I. 26100, C.I. 60725, C.I. 12150, C.I. 75810, C.I. 75300.

The addition of a dye has the advantage that it provides of a visible indication for the user, sending the message of particular (active) ingredients having been incorporated in the lipid phase. It allows furthermore to visualize the stability of the phase, in particular of the lipid phase, that has been applied on the sheet can be easily visualized. This allows, for example, to monitor whether the oily and aqueous phases have become mixed upon the storage.

### Emulsifiers

The lipid and/or aqueous phase in the products of the invention may further contain one or more emulsifiers which can be of the W/O (for use in the lipid phase) or the O/W (for use in the aqueous phase) type. The addition of an emulsifier allows the incorporation of hydrophilic components or agents into the lipid phase and vice versa of lipophilic components or agents into the aqueous phase.

Preferred are non-ionic emulsifiers which typically have good skin compatibility. Improved sensoric properties are obtained when combining non-iononics W/O and O/W emulsifiers. The lipid and/or aqueous phase may contain the emulsifier(s) in an amount of 0 to 20 % (w/w), respectively 0.1 to 15 % (w/w) and in particular 0.1 to 10 % (w/w) relative to the total quantity of the lipid and/or aqueous phase.

### Non-ionic emulsifiers

Particular non-ionic emulsifiers comprise:
(1) Addition products of 2 to 50 moles of ethylene oxide and/or 0 to 20 moles propylene oxide to linear fatty alcohols having 8 to 40 C-atoms, to fatty acids with 12 to 40 C-atoms and to alkylphenols with 8 to 15 C-atoms in the alkyl rest.
(2) C_{12/18}-fatty acid mono- and -diesters of addition products of 1 to 50 moles of ethylene oxide and glycerine.
(3) Glycerine mono- and -diesters and sorbitan mono- and -diesters of saturated and unsaturated fatty acids with 6 to 22 C-atoms and their ethylene oxide addition products.
(4) Alkyl mono- and -oligoglycosides with 8 to 22 C-atoms in the alkyl rest and their ethoxylated analogs.
(5) Addition products of 7 to 60 moles of ethylene oxide to castor oil and/or hardened castor oil.
(6) Polyol- and in particular polyglycerine esters, such as e.g. polyol poly-12-hydroxystearate, polyglycerine polyricinoleate, polyglycerine diisostearate or polyglycerine dimerate. Also applicable are mixtures of compounds of several of these substance classes.
(7) Addition products of 2 to 15 moles of ethylene oxide to castor oil and/or hardened castor oil.
(8) Partial esters derived from linear, branch chained, unsaturated or saturated C₆-C₂₂-fatty acids, ricinoleic acid as well as 12-hydroxystearic acid and glycerine, polyglycerine, pentaerythrite, dipentaerythrit, sugar alcohols (e.g. sorbitol), alkylglucosides (e.g. methylglucoside, butylglucoside, laurylglucoside) as well as polyglucosides (e.g. cellulose), or mixed esters such as e.g. glyceryl stearate/citrate and glyceryl stearate/lactate.
(9) Wool wax alcohols.
(10) Polysiloxane-polyalkyl-polyether-copolymers and derivatives thereof.
(11) Mixed esters from pentaerythrite, fatty acids, citric acid and fatty alcohols and/or mixed esters of fatty acids with 6 to 22 C-atoms with methylglucose and polyoles, respectively glycerine or polyglycerine.
(12) Polyalkylene glycols.

The addition products of ethylene oxide and/or of propylene oxide and fatty alcohols, fatty acids, alkylphenoles, glycerine mono- and -diesters as well as sorbitan mono- and
- diesters of fatty acids or of castor oil are known and commercially available products. Usually these are mixtures of homologues of which the average degree of alkoxylation corresponds to the ratio of starting quantities of ethylene oxide and/or propylene oxide and substrate, with which the addition reaction is conducted. Depending upon the degree of alkoxylation these products are either W/O- or O/W-emulsifiers. C_{12/18}-fatty acid mono- and -diesters of addition products of ethylene oxide to glycerine are known as re-fatting agents in cosmetic applications.

Particular useful and mild emulsifiers are polyolpoly-12-hydroxystearates and mixtures thereof with other components, that are available under the tradename "Dehymuls^{®} PGPH" (W/O-emulsifier) or "Eumulgin^{®} VL 75" (1:1 w/w mixture with coco-glucosides, O/W-emulsifier) or Dehymuls^{®} SBL (W/O-Emulsifier) from Cognis Deutschland GmbH. The polyol components of these emulsifiers can be derived from materials that have at least two and in particular 3 to 12 and more in particular 3 to 8 hydroxyl groups, and 2 to 12 carbon atoms.

In case it is desirable to incorporate water-soluble active ingredients and/or small amounts of water into the lipid phase it can be advantageous to add an emulsifier selected from from the group of non-ionic O/W-emulsifiers (HLB-value: 8 - 18) and/or solubilizers. These can for example be the already mentioned ethylene oxide-adducts with a corresponding high degree of ethoxylation e.g. 10 - 20 ethylene oxide units in the case of O/W-emulsifiers and 20 - 40 ethylene oxide units for so-called solubilizers. Particularly attractive as O/W emulsifiers are Ceteareth-12 und PEG-20 stearate. Particularly attractive solubilizers are Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether) and Eumulgin^{®} SML 20 (INCI: Polysorbate-20).

Non-ionic emulsifiers of the group of alkyl oligoglycoside are particularly skin-compatible and therefore preferred as O/W-Emulsifiers. C₈-C₂₂-alkyl mono- and - oligoglycosides, their preparation and use have been described in the prior art. Oligoglycosides are meant to comprise oligomeric glycosides with a degree of oligomerisation of up to about 8. The degree of oligerisation can also be a statistical average used for those products comprised of a specific range of oligoglycosides. An example is the product sold under the tradename Plantacare^{®} which has a C₈-C₁₆-alkyl group glycosidically bound to an oligoglucoside rest, having an average degree of oligomerisation between 1 and 2.

Other non-ionic emulsifiers are the acyl glucamides. Preferred is the product sold under the tradename Emulgade^{®} PL 68/50 (Cognis Deutschland GmbH) which is a 1:1-mixture of alkyl polyglucosides and fatty alcohols, and a mixture of lauryl glucoside, polyglyceryl-2-dipolyhydroxystearate, glycerine and water, sold under the trade name Eumulgin^{®} VL 75.

Lipophilic W/O-emulsifiers in principle are emulsifiers with a HLB-value in the range of 1 to 8, that are described, for example, in Kirk-Othmer, "Encyclopedia of Chemical Technology", 3rd Ed., 1979, Vol. 8, p. 913. The HLB-value of ethoxylated products is calculated by the formula: HLB = (100 - L) : 5, wherein L is the percentage (in weight %) of lipophilic groups, i.e. of fatty alkyl- or fatty acyl groups in the ethylene oxide adducts.

Particularly attractive W/O-emulsifiers are the partial esters of polyoles, in particular of mono-, di- or tri-, sesqui esters of fatty acids of polyoles, more in particular of C₃-C₆-polyoles, such as, for example, glyceryl monoesters, partial esters of pentaerythrite or carbohydrate esters, e.g. saccharose distearate, or sorbitane mono-, di-, tri- or sesqui fatty esters in particular stearates, oleates, erucates, ricinoleates, hydroxystearates, isostearates (but also: tartrates, citrates, maleates) and the like. Also attractive are addition products of 1 to 30, respectively 5 to 10 moles ethylene oxide to these sorbitane esters.

### Further Surfactants/Emulsifiers for both phases

Depending upon the use of the products of the present invention, the lipid and/or aqueous phase may further contain zwitterionic, amphoteric, cationic and or anionic surfactants.

Zwitterionic surfactants are those tensioactive compounds, that contain at least a quaternary ammonium group and at least a -COO⁽⁻⁾- or -SO₃⁽⁻⁾- group. Particularly useful zwitterionic surfactants are the so-called betaines such as N-alkyl-N,N-dimethyl ammonium glycinate, for example coco-alkyl dimethylammonium glycinate, N-acyl-aminopropyl-N,N-dimethylammonium glycinate, for example coco-acyl aminopropyl dimethylammonium glycinate, and 2-alkyl-3-carboxylmethyl-3-hydroxyethylimidazoline, each having 8 to 18 C-atoms in the alkyl- or acyl group as well as coco-acyl aminoethyl hydroxyethyl carboxymethyl glycinate. A preferred zwitterionic surfactant is the fatty acid amide-derivative known by its INCI-name cocamidopropyl betaine.

Ampholytic surfactants can further be added, in particular as co-surfactants. Ampholytic surfactants are understood to comprise those tensioactive compounds, that beside a C₈-C₁₈-alkyl- or acyl group at least contain a free amino group and at least a - COOH- or -SO₃H- group and are able to form internal salts. Examples of appropriate ampholytic surfactants are N-alkyl glycines, N-alkyl propionic acids, N-alkyl amino buteric acids, N-alkyl iminodipropionic acids, N-hydroxyethyl-N-alkyl amidopropyl glycines, N-alkyl taurine, N- alkyl sarcosine, 2-alkylaminopropionic acids and alkylamino acetic acids with in each alkyl group about 8 to 18 C-atoms.

Most preferred ampholytic surfactants N-coco-alkyl aminopropionate coco-acyl amino ethylamino propionate and C₁₂₋₁₈-acylsarcosine.

Anionic surfactants are characterized by a water solubilizing anionic group such as a carboxylate-, sulfate-, sulfonate- or phosphate- group and a lipophilic rest. Particular anionic surfactants are the alkali-, ammonium- or alkanol ammonium salts of alkyl sulfates, alkyl ethersulfates, alkyl ethercarboxylates, acyl isethionates, acyl sarkosinates, acyl taurines with linear alkyl- or acyl groups having 12 to 18 C-atoms as well as alkali- or ammonium salts of sulfosuccinates and acyl glutamates.

Quaternary ammonium derivatives can in particular be used as cationic surfactants. Preferred are ammonium halogenides, in particular chlorides and bromides, e.g. alkyl trimethylammonium chloride, dialkyl dimethylammonium chloride and trialkyl methylammonium chloride, z. B. cetyl trimethylammonium chloride, stearyl trimethylammonium chloride, distearyl dimethylammonium chloride, lauryl dimethylammonium chloride, lauryl dimethylbenzylammonium chloride and tricetyl methylammonium chloride. Additional cationic surfactants are the quaternary esters with good biological degradability, such as, for example, dialkylammonium methosulfates and methylhydroxyalkyl dialkoyloxy alkylammonium methosulfates (sold under the tradename Stepantex^{®} and the products of the Dehyquart^{®}-series). The term "Esterquats" is meant to comprise quaternized fatty acid triethanolamine ester salts which have a beneficial impact on the softness of the phases, in particular of the lipid phase. Further cationic surfactants are the quaternized protein hydrolysates.

### Application and properties

The products according to the present invention advantageously result in an optimal release of the active ingredient(s), in particular when incorporated in the lipid phase, onto the skin during use.

Optimal release of active ingredients can be achieved by using a lipid phase which is a solid lipid having a melting point or melting range which is equal to or slightly exceeds body temperature. Without being bound to theory, it is believed that this results in a quicker melting of the lipid phase causing a faster and more efficient transfer and release to the skin of the active materials.

Optimal release of active ingredients can also be achieved by using a suitable emulsifier in one or both of the phases to cause a local emulsification process on the skin during use of the wipes. Preferably the emulsifier is present in the aqueous phase. This local emulsification may be the result of body temperature causing the lipid phase to melt or it may be the result of pressure exerted during usage of the wipe, or it may be the result of both, the latter being usually the case. In the instance of local emulsification by the effect of pressure, the emulsification process is driven by the (limited) pressure exerted by the user when applying the wipe, e.g. by rubbing it across the skin, dabbing it and the like. This causes the two phases to contact and form an emulsion locally.

In this local emulsification process, a limited amount of the phase without emulsifier is incorporated into the phase having the emulsifier. In a preferred embodiment, the aqueous phase contains a small amount of emulsifier, for example the emulsifier may be present in an amount from about 0.5 to about 5%, more in particular from about 1 to about 3%. In that instance some of the lipid phase is locally emulsified into the aqueous phase.

Although in preferred executions the lipid phase is not present on the whole surface of the wipe, good release of the lipid phase and of the components contained therein is attained, in particular when the local emulsification process comes into play.

Optimal release of active ingredients can also be achieved by making use of both above possibilities.

### Manufacture.

This invention futher concerns a process for preparing a product as defined herein, said process comprising contacting a porous or absorbent sheet with a lipid phase composition and an aqueous phase composition as described herein. The porous or absorbent sheet in particular is made of a non-woven material. The process comprises contacting the sheett simultaneously or subsequently with the lipid phase and the aqueous phase.

In a particular execution, the process comprises contacting the sheet with a lipid phase and subsequently with an aqueous phase.

The lipid and aqueous phases can be applied to the sheet at any time during the manufacturing process of the sheet, for example either one or both of the phases may be applied during the manufacturing process of the sheet material. Preferably the lipid and/or aqueous phase can be applied to the sheet after finishing the manufacturing process of the sheet, more preferably after the sheet has been dried.

The lipid phase may also be applied to the sheet material just after its manufacture while still being wet.

In a particular execution, the carrier material is cut into strips, the transversal size of which being similar to the size of the tissue, wipe or towelette. Subsequently, the lipid and aqueous phases are applied to these strips, preferably first the lipid phase and then the aqueous phase. Thereafter the strips are folded according to methods generally known and applied in the art. In an alternative execution, the lipid phase is applied to these strips, which are subsequently folded and the thus folded strips are moistened with the aqueous phase as, said moistening preferably comprising spraying or drippling, or by immersing in or running the strip through a bath containing the aqueous phase. The latter can also be sprayed or printed onto the strips.

In a further step, the strips are cut so that the desired size of the sheet, in particular of the wipes, is obtained. The thus obtained sheets (or wipes) can be packed individually or can be stacked in a determined number, e.g. a number between 10 and 30, preferably between 15 and 25, most preferably about 20, or a number between 50 and 100, preferably between 60 and 80, most preferably about 72, and the stack then packed in a suitable package, for example a plastic wrap, box and the like.

Wipes with different coating and/or impregnation can be combined in one packaging. For example there can be a stack of wipes with increasing or decreasing amounts of lipid phase. Or colored or uncolored wipes can be alternated, e.g. there can be provided a pack of wipes where every fifth wipe has colored stripes.

The products according to the invention can take the form of baby or adult wipes and can be used in a wide range of applications as personal care products, comprising, for example, baby cleansing wipes, face or body cleansing wipes, wipes for skin treatment or skin conditioning such as for example skin moisturization and against skin aging, insect repellent wipes, powder wipes, toilet wipes, anti-perspirant wipes, peeling wipes, after-sun treatment wipes, sunscreen wipes, wipes for feminine hygiene, nappy rash wipes, the latter preferably containing zinc oxide as active ingredient, and the like.

The products of the invention may find use as cleansing tools, however their use is not limited to this application only. They have been found to be more effective cleansers compared to products that have only an aqueous phase. This is due, i.a., by the fact that they can remove both aqueous and lipid soils and components.

The products described herein find use as sheets of active substances, in particular of the active substances mentioned herein, or they find use as both cleanser and sheet of active substances in one product.

Traditionally, wipes have been used primarily as a cleansing tool. Applications as a vehicle for active substances, i.e. so-called leave-on products, have been limited because of the poor transfer rate of the active ingredients from the wipe to the skin. The products of this invention provide a solution to this problem in that they result in an excellent transfer of active ingredients to the skin thus widening the applications of wipe products as a vehicle for a number of actives, in particular more expensive actives that so far could not be applied through wipes because of poor transfer rate. The products of this invention not only provide a more efficient transfer of active ingredients to the skin, but moreover provide other consumer benefits such as a more even distribution of the actives on the skin, better skin penetration.

The products of this invention show the additional advantage that they may combine in one and the same product both cleansing capability and the transfer of active ingredients to the skin, i.e. the application of leave-on products.

Either of both aspects may be present in a larger extend, i.e. the product may be primarily for cleansing purposes but also having the capability of transferring certain beneficial components or active substances to the skin, or vice versa, the products may be designed for applications in instances where the primary benefit is not cleansing but a better and more convenient form of application of leave-on products. Hence the products of this invention allow the possibility to be optimized against two key consumer needs - cleansing and caring.

The products of the invention therefore show improved performance in terms of cleansing and skin benefits since both benefits can be formulated in different phases independently.

Another benefit of the products of this invention is that they offer a softer feel of the fabric due to the modification of the fabric surface caused by the presence of the lipid phase. The products moreover offer gentler cleansing because of less friction of the wipe on the skin (softer skin-feel).

As used herein, softness refers to the tactile sensation perceived when the consumer holds the product, rubs it across the skin, or crumples it with the hand.

The products of this invention additionally offer the possibility to incorporate into or apply to one product two or more incompatible ingredients, thus allowing the user to apply incompatible agents with one and the same product. In particular it is possible to have a product that has as well water soluble as lipid soluble ingredients, for example a wipe that has both active ingredients that are water soluble and oil soluble.

A still further advantage lies in the fact that the instant products allow an improved transfer of actives onto the skin since the active ingredients are concentrated on the surface of the sheet / fabric material and not included in the inner phase of a typical o/w-emulsion.

The products according to the invention, and in particular the particular compositions of the lipid and aqueous phase described in this specification, possess the additional advantage that they are almost odorless (unless fragrances are added), environmentally friendly and biologically decomposable.

The products of this invention are particularly attractive because they allow convenient and quick application (easy to carry), and an easier and more evenly distribution of the product. They moreover are easy to apply on babies and children. The products are more efficient in that they allow faster cleansing.

In view of these beneficial properties, the products of this invention can be used in a wide variety of cosmetic and personal care applications, but also in other cleaning or cleansing applications such as cleaning of hard surfaces.

### Examples

The following examples are given with the nomenclature of INCI. As used in the following examples, C.I. refers to dyes.

### Example 1: lipid phases

### Phase 1-A

| | |
|---|---|
| Cocoglycerides | 64.99 % |
| Cetyl Alcohol | 33.00 % |
| Di-Stearyl Ether | 1.00 % |
| Tocopherol | 1.00 % |
| C.I. 61565 | 0.01 % |

### Phase 1-B

| | |
|---|---|
| Cocoglycerides | 54.99 % |
| Cetyl Alcohol | 33.00 % |
| Ceteareth-12 | 3.00 % |
| Glyceryl Stearate | 4.00 % |
| Di-Stearyl Carbonate | 2.00 % |
| Tocopherol | 1.00 % |
| C.I. 61565 | 0.01 % |
| Aqua | 2.00 % |

### Phase 1-C

| | |
|---|---|
| Cocoglycerides | 49.99 % |
| Cetearyl Alcohol | 20.00 % |
| Cegesoft® HF 52 | 5.00 % |
| Cegesoft® PS 6 | 3.00 % |
| Ceteareth-12 | 2.00 % |
| Glyceryl Stearate | 2.00 % |
| PEG-20 Stearate | 10.00 % |
| Di-Stearyl Ether | 2.00 % |
| Tocopherol | 1.00 % |
| C.I. 61565 | 0.01 % |
| Aqua | 5.00 % |

### Phase 1-D

| | |
|---|---|
| Cocoglycerides | 58.99 % |
| Glyceryl Stearate | 25.00 % |
| Glyceryl Laurate | 14.00 % |
| Di-Stearyl Carbonate | 1.00 % |
| Tocopherol | 1.00 % |
| C.I. 75300 | 0.01 % |

### Phase 1-E

| | |
|---|---|
| Cocoglycerides | 30.00 % |
| Cetearyl Alcohol | 1.00 % |
| Cegesoft® HF 52 | 20.00 % |
| Cegesoft® GPO | 5.00 % |
| Ceteareth-12 | 15.00 % |
| Glyceryl Stearate | 20.00 % |
| Di-Stearyl Ether | 5.00 % |
| Tocopherol | 1.00 % |
| Panthenol | 1.00 % |
| Aqua | 2.00 % |

### Phase 1-F

| | |
|---|---|
| Cocoglycerides | 19.99 % |
| Cetearyl Alcohol | 30.00 % |
| Cegesoft® PS 6 | 10.00 % |
| Eumulgin® VL 75 | 10.00 % |
| Ceteareth-12 | 5.00 % |
| Glyceryl Stearate | 10.00 % |
| Di-Stearyl Carbonate | 5.00 % |
| Tospearl® 145 A | 5.00 % |
| Zinc Stearate | 2.00 % |
| C.I. 61565 | 0.01 % |
| Aqua | 3.00 % |

### Phase 1-G

| | |
|---|---|
| Myristyl Alcohol | 19.99 % |
| Cocoglycerides | 10.00 % |
| Cegesoft® HF 52 | 20.00 % |
| Eumulgin® VL 75 | 10.00 % |
| Glyceryl Stearate | 20.00 % |
| PEG-20 Stearate | 5.00 % |
| Di-Stearyl Carbonate | 2.00 % |
| Panthenol | 3.00 % |
| C.I. 61565 | 0.01 % |
| Aqua | 10.00 % |

### Phase 1-H

| | |
|---|---|
| Myristyl Alcohol | 47.99 % |
| Stearyl Alcohol | 25.00 % |
| Eumulgin® VL 75 | 2.00 % |
| PEG-20 Stearate | 14.00 % |
| 1,2-Hexadecanediol | 5.00 % |
| Bisabolol | 1.00 % |
| C.I. 47000 | 0.01 % |
| Aqua | 5.00 % |

### Phase 1-I

| | |
|---|---|
| Cocoglycerides | 47.99 % |
| Stearyl Alcohol | 20.00 % |
| Eumulgin® VL 75 | 2.00 % |
| PEG-20 Stearate | 12.00 % |
| Di-Stearyl Carbonate | 5.00 % |
| Cyclomethicone | 3.00 % |
| Tospearl® 145 A | 5.00 % |
| C.I. 75300 | 0.01 % |
| Aqua | 5.00 % |

### Phase 1-J

| | |
|---|---|
| Cocoglycerides | 55.99 % |
| Glyceryl Stearate | 20.00 % |
| Glyceryl Laurate | 15.00 % |
| Di-Stearyl Carbonate | 5.00 % |
| Talc | 2.00 % |
| Aluminum Starch Octenylsuccinate | 2.00 % |
| C.I. 60725 | 0.01 % |

### Phase 1-K

| | |
|---|---|
| Cocoglycerides | 50.99 % |
| Glyceryl Stearate | 25.00 % |
| Glyceryl Laurate | 15.00 % |
| Di-Stearyl Ether | 5.00 % |
| Talc | 2.00 % |
| Timiron® Splendid Gold | 2.00 % |
| C.I. 21230 | 0.01 % |

### Phase 1-L

| | |
|---|---|
| Myristyl Alcohol | 58.99 % |
| Stearyl Alcohol | 23.00 % |
| PEG-20 Stearate | 15.00 % |
| Di-Stearyl Carbonate | 2.00 % |
| Panthenol | 1.00 % |
| C.I. 61525 | 0.01 % |

### Phase 1-M

| | |
|---|---|
| Myristyl Alcohol | 47.99 % |
| Stearyl Alcohol | 25.00 % |
| Eumulgin® VL 75 | 2.00 % |
| PEG-20 Stearate | 10.00 % |
| Di-Stearyl Ether | 7.00 % |
| Panthenol | 2.00 % |
| C.I. 61525 | 0.01 % |
| Aqua | 6.00 % |

### Phase 1-N

| | |
|---|---|
| Myristyl Alcohol | 50.00 % |
| Stearyl Alcohol | 25.00 % |
| Eumulgin® VL 75 | 2.00 % |
| PEG-20 Stearate | 10.00 % |
| Di-Stearyl Ether | 7.00 % |
| Ethyl Butylacetylaminopropionate | 5.00 % |
| Panthenol | 1.00 % |

### Phase 1-O

| | |
|---|---|
| Cocoglycerides | 54.99 % |
| Cetyl Alcohol | 33.00 % |
| Ceteareth-12 | 3.00 % |
| Glyceryl Stearate | 4.00 % |
| Di-Stearyl Carbonate | 2.00 % |
| Octyl Methoxycinnamate | 6.00 % |
| C.I. 61565 | 0.01 % |

### Phase 1-P

| | |
|---|---|
| Cocoglycerides | 56.99 % |
| Glyceryl Stearate | 25.00 % |
| Glyceryl Laurate | 14.00 % |
| Di-Stearyl Carbonate | 1.00 % |
| Polyethylene | 3.00 % |
| C.I. 75300 | 0.01 % |

### Phase 1-Q

| | |
|---|---|
| Cocoglycerides | 58.93 % |
| Glyceryl Stearate | 25.00 % |
| Glyceryl Laurate | 15.00 % |
| Di-Stearyl Ether | 1.00 % |
| Aqua | 0.06 % |
| C.I. 61565 | 0.01 % |

### Phase 1-R

| | |
|---|---|
| Cocoglycerides | 43.93 % |
| Stearyl Alcohol | 15.00 % |
| Glyceryl Stearate | 25.00 % |
| Glyceryl Laurate | 15.00 % |
| Di-Stearyl Ether | 1.00 % |
| Aqua | 0.06 % |
| C.I. 61565 | 0.01 % |

### Phase 1-S

| | |
|---|---|
| Cocoglycerides | 44.93 % |
| Glyceryl Stearate | 25.00 % |
| Glyceryl Laurate | 15.00 % |
| Di-Stearyl Ether | 15.00 % |
| Aqua | 0.06 % |
| C.I. 61565 | 0.01 % |

### Example 2: aqueous phases

### Phase 2-A

| | |
|---|---|
| Aqua | 96.336 % |
| Polysorbate 20 | 0.600 % |
| PEG-75 Lanolin | 0.100 % |
| Perfume | 0.150 % |
| PEG-40 Hydrogenated Castor Oil | 0.400 % |
| Propylene Glycol | 1.120 % |
| Phenoxyethanol | 0.800 % |
| Tetrasodium EDTA | 0.078 % |
| Chamomilla Recutita | 0.070 % |
| Ethoxydiglycol | 0.171 % |
| Butylene Glycol | 0.035 % |
| Glucose | 0.016 % |
| Iodopropynyl Butylcarbamate | 0.010 % |
| PEG- 4 Laurate | 0.090 % |
| Citric Acid | 0.020 % |

### Phase 2-B

| | |
|---|---|
| Aqua | 98.252 % |
| Phenoxyethanol | 0.800 % |
| Iodopropynyl Butylcarbamate | 0.010 % |
| PEG- 4 Laurate | 0.090 % |
| Perfume | 0.150 % |
| Tetrasodium EDTA | 0.078 % |
| Citric Acid | 0.020 % |
| Polysorbate 20 | 0.600 % |

### Phase 2-C

| | |
|---|---|
| Aqua | 97.250 % |
| Glycerines | 1.000 % |
| Phenoxyethanol | 0.800 % |
| Iodopropynyl Butylcarbamate | 0.010 % |
| PEG- 4 Laurate | 0.090 % |
| Perfume | 0.150 % |
| Tetrasodium EDTA | 0.078 % |
| Citric Acid | 0.020 % |
| Polysorbate 20 | 0.600 % |

### Phase 2-D

| | |
|---|---|
| Aqua | 96.332 % |
| Glycerines | 1.000 % |
| Phenoxyethanol | 0.800 % |
| Polysorbate 20 | 0.600 % |
| PPG-15 Stearyl Ether | 0.400 % |
| PEG-7 Glyceryl Cocoate | 0.100 % |
| Propylene Glycol | 0.350 % |
| Iodopropynyl Butylcarbamate | 0.010 % |
| PEG- 4 Laurate | 0.090 % |
| Chamomilla Recutita | 0.070 % |
| Perfume | 0.150 % |
| Tetrasodium EDTA | 0.078 % |
| Citric Acid | 0.020 % |

### Phase 2-E

| | |
|---|---|
| Aqua | 97.33 % |
| Phenoxyethanol | 0.800 % |
| Polysorbate 20 | 0.600 % |
| Sorbeth-30 | 0.400 % |
| Propylene Glycol | 0.350 % |
| Dimethicone Copolyol | 0.100 % |
| Iodopropynyl Butylcarbamate | 0.010 % |
| PEG- 4 Laurate | 0.090 % |
| Chamomilla Recutita | 0.070 % |
| Perfume | 0.150 % |
| Tetrasodium EDTA | 0.078 % |
| Citric Acid | 0.020 % |

### Phase 2-F

| | |
|---|---|
| Aqua | 97.332 % |
| Phenoxyethanol | 0.800 % |
| PEG-80 Sorbitan Laurate | 0.600 % |
| Propylene Glycol | 0.350 % |
| Sorbeth-30 | 0.400 % |
| Octyldecanol | 0.100 % |
| Iodopropynyl Butylcarbamate | 0.010 % |
| PEG-4 Laurate | 0.090 % |
| Chamomilla Recutita | 0.070 % |
| Perfume | 0.150 % |
| Tetrasodium EDTA | 0.078 % |
| Citric Acid | 0.020 % |

### Phase 2-G

| | |
|---|---|
| Aqua | 97.332 % |
| Phenoxyethanol | 0.800 % |
| Polysorbate-20 | 0.600 % |
| PGG-15 Stearyl Ether | 0.400 % |
| Propylene Glycol | 0.350 % |
| Decyl Oleate | 0.100 % |
| Iodopropynyl Butylcarbamate | 0.010 % |
| PEG-4 Laurate | 0.090 % |
| Chamomilla Recutita | 0.070 % |
| Perfume | 0.150 % |
| Tetrasodium EDTA | 0.078 % |
| Citric Acid | 0.020 % |

### Phase 2-H

| | |
|---|---|
| Sodium Myreth Sulfate | 10.00 % |
| Lauryl Glucoside | 15.00 % |
| Cocamidopropyl Betaine | 10.00 % |
| Aqua | 64.50 % |
| Perfume | 0.50 % |

### Phase 2-I

| | |
|---|---|
| Sodium Laureth Sulfate | 20.00 % |
| Decyl Glucoside | 5.00 % |
| Cocamidopropyl Betaine | 8.00 % |
| Laureth-2 | 2.50 % |
| Polysorbate-20 | 1.00 % |
| Aqua | 63.00 % |
| Perfume | 0.50 % |

### Phase 2-J

| | |
|---|---|
| Sodium Myreth Sulfate | 15.00 % |
| Lauryl Glucoside | 10.00 % |
| Laureth-2 | 1.50 % |
| Aqua | 73.00 % |
| Perfume | 0.50 % |

### Phase 2-K

| | |
|---|---|
| Emulgade® CM | 20.00 % |
| Polysorbate 20 | 0.80 % |
| Coco-Glucoside | 2.50 % |
| Phenoxyethanol | 1.00 % |
| Cetylpyridinium Cloride | 0.10 % |
| Tetrasodium EDTA | 0.20 % |
| Aqua | 75.22 % |
| Citric Acid | 0.08 % |
| Perfume | 0.10 % |

### Phase 2-L

| | |
|---|---|
| Emulgade® SE-PF | 1.66 % |
| Ceteareth-12 | 0.94 % |
| Lamesoft® PO 65 | 0.25 % |
| Paraffinum Liquidum | 3.00 % |
| Cetylpyridinium Cloride | 0.05 % |
| Polysorbate-20 | 1.00 % |
| Citric Acid | 0.03 % |
| Tetrasodium EDTA | 0.20 % |
| Nipaguard® IPF | 0.10 % |
| Aqua | 92.66 % |
| Perfume | 0.11 % |

### Phase 2-M

| | |
|---|---|
| Emulgade® SE-PF | 1.627 % |
| Ceteareth-12 | 0.921 % |
| Lamesoft® PO 65 | 0.245 % |
| Paraffinum Liquidum | 2.940 % |
| Glyceryl Polymethacrylate | 2.000 % |
| Cetylpyridinium Cloride | 0.049 % |
| Polysorbate-20 | 0.980 % |
| Citric Acid | 0.029 % |
| Tetrasodium EDTA | 0.196 % |
| Nipaguard® IPF | 0.098 % |
| Aqua | 90.807 % |
| Perfume | 0.108 % |

### Example 3

Dry hydro-entangled sheet material made of fabric having a surface weight of 50 g/m2 was cut into strips. The lipid phase, prepared as set forth in example 1, was applied onto both sides of the fabric with the total amount of 1.0 g in the form of stripes by using the contact process. This comprised running the fabric strips against two heated heads having a slitted blade each mounted on one side of the fabric strip. The lipid phase on the strips was allowed to cool so that it solidified and the strips were subsequently sprayed in the conventional manner with the liquid as prepared in example 2. Liquid addition was set at 6 g per wipe. Subsequently the strips were folded and cut.

## Claims

1. A product comprising a porous or absorbent sheet for use as a combined cleanser and applicator for active substances whereto an aqueous and a lipid phase, which has a melting point or melting range of above or equal to 25°C, have been applied, wherein the lipid phase comprises mono-, di- or triglycerides and/or fatty alcohols, and wherein the lipid and/or the aqueous phase contains one or more active substances.

2. A product according to claim 1 wherein the melting point or melting range of the lipid phase is in the range of 32 to 40°C.

3. A product according to claim 1 wherein the lipid phase comprises mono-, di- or triglycerides derived from or present in natural oils.

4. A product according to claim 1 wherein the lipid phase comprises fatty acid mono-, di- or triglycerides wherein the fatty acids contain from 12 to 24, preferably from 16 to 20 carbon atoms.

5. A product according to claim 1 wherein the lipid phase comprises triglycerides selected from glyceryl stearate, glyceryl oleate, glyceryl laurate, glyceryl myristate, cocoglycerides, or hydrogenated palm oil glycerides, hydrogenated castor oil, or hydrogenated rapeseed oil.

6. A product according to any of the preceding claims wherein the lipid phase comprises mono-, di- or triglycerides in an amount of at least 50 %, preferably at least 70 %, more preferably at least 90 %, w/w of the total amount of components making up the lipid phase.

7. A product according to claim 1 wherein the lipid phase contains C₁₂-C₅₀-fatty alcohols, in particular the C₁₂-C₂₄-fatty alcohols.

8. A product according to claim 7 wherein the fatty alcohols are selected from myristyl alcohol, 1-pentadecanol, cetyl alcohol, lauryl alcohol, oleyl alcohol, palmityl alcohol, 1-heptadecanol, stearyl alcohol, cetearyl alcohol, 1-nonadecanol, arachidyl alcohol, 1-heneicosanol, behenyl alcohol, brassidyl alcohol, lignoceryl alcohol, ceryl alcohol or myricyl alcohol and C₁₆/C₁₈-Guerbet alcohols.

9. A product according to any of the preceding claims wherein the fatty alcohols are present in the lipid phase, in an amount relative to the total weight amount of the lipid phase, which is in the range of 1 - 40 %, preferably 1 - 30 % (w/w), more preferably of 1 - 20 % (w/w), still more preferably from 1 -10 % (w/w).

10. A product according to claim 1 or 2 wherein the lipid phase further contains fatty acids.

11. A product according to claim 10 wherein the fatty acids are C₁₄-C₄₀-fatty acids or in particular are C₁₆-C₃₀-fatty acids.

12. A product according to claim 10 wherein the fatty acids are selected from myristic-, pentadecanoic-, palmitic-, margaric-, stearic-, nonadecanoic-, arachic-, behenic-, lignoceric-, cerotic-, melissic-, erucaic-, elaeostearic-, oleic-, linoleic-, lauric acid and hydroxy-substituted fatty acids.

13. A product according to any of claims 10 to 12 wherein the total amount of the fatty acids present in the lipid phase, relative to the total weight amount of the lipid phase, is in the range of 1 - 30 % (w/w), preferably of 1 - 20 % (w/w), more preferably from 1 -10 % (w/w).

14. A product according to claim 1 or 2 wherein the lipid phase contains one or more of components (a), (b), (c), (d), (e) or (f) as defined hereafter:
(a) at least 1 - 50 % (w/w), in particular at least 1 - 10 % of an oily or waxy component
(b) 0,1 - 5 % (w/w) of at least one active ingredient
(c) 1 - 10 % (w/w) of at least one oil
(d) 0.1 - 10 % (w/w) of at least one emulsifier
(e) 5 - 90 % (w/w) of further waxy components
(f) 0 - 5 % (w/w) water.

15. A product according to claim 14 wherein the lipid phase contains all components (a)-(f).

16. A product according to claim 1 wherein the active substance(s) is or are anti-microbials, e.g. anti-bacterials and antifungals, anti-inflammatory agents, anti-irritating, anti-itching, anti-perspirant agents.

17. A product according to any of claims 1 to 15 wherein the lipid or the aqueous phase contains at least one moisturizer, deodorant, skin caring ingredient, plant extract, vitamin, perfume oil, dye, sunscreen filter, hydrotrope or self-tanning agent.

18. A product according to any of claims 1 to 15, wherein the lipid or the aqueous phase contains at least one emulsifier.

19. A product according to any of claims 1 to 15, wherein the lipid phase contains at least one superfatting agent, thickener, cationic polymer, aniomic polymer, zwitterionic polymer, amphoteric polymer, consistency agent, anti-oxidant.

20. A product according to any of claims 1 to 15 wherein the lipid or the aqueous phase contains an insect repellent, a sunscreen filter, a powder or a peeling agent.

21. A product according to any of claims 1 to 20 which is a wipe.

22. A product according to claim 21 wherein the wipe is a non-woven wipe.

23. A method of manufacturing a product as claimed in any of claims 1 to 22 said method comprising contacting the sheet with a lipid phase and with an aqueous phase, either subsequently or simultaneously.

24. A method according to claim 23 wherein a lipid phase having a melting point or a melting range of above room temperature is first applied to the surface of the sheet and subsequently the aqueous phase is applied.

25. A method according to claim 23 wherein the the aqueous phase by spraying, drippling, immersing or running through a bath, and the lipid phase is applied by spraying, contacting, printing or a direct contact process where there is a direct contact between the sheet and an application head having slit nozzles.

26. Use of a product as claimed in any of claims 1 to 22 as a combined cleanser and sheet of active substances.
